# EUROPEAN PATENT APPLICATION

(11) **EP 3 670 647 A1**
(43) Date of publication of application: **24.06.2020**
(21) Application number: 18382933.2
(22) Date of filing: 17.12.2018
(51) Int. Cl.: C12N 1/20, C12R 1/40, A01N 63/00, A01N 63/02, C12N 15/01, C12P 13/22, C12P 13/12, C12P 13/10

(54) **STRAINS CAPABLE OF PRODUCING PLANT GROWTH-STIMULATING COMPOUNDS**

(71) Applicant: Bio-Iliberis Research and Development S.L., 18210 Peligros, Granada (ES)
(72) Inventor: Roca Hernández, Amalia, 18210 Peligros, Granada (ES); PIZARRO TOBÍAS, Paloma, 18210 Peligros, Granada (ES)
(74) Representative: ABG Intellectual Property Law, S.L.

(57) **Abstract**

The invention relates to a variant strain of *Pseudomonas putida* BIRD-1 with accession number CECT 7415, which variant produces at least one plant growth stimulating compound and/or solubilises insoluble phosphate and/or iron, and to methods and uses thereof. The preferred growth stimulating compound is one or more amino acids that favour phytohormone biosynthesis, enhance endurance against abiotic stress and favour nitrogen feeding in plants. The invention also relates to fertilisers, seeds, root balls, solid supports, soils or fields comprising the variant strain, and to methods and uses thereof.

## Description

### FIELD OF THE INVENTION

The invention relates to the field of microbiological strains having the capacity to produce at least one plant growth stimulating compound and/or to solubilise insoluble phosphate and/or iron and methods and their applications. The invention also relates to methods to obtain such plant growth promoting strains.

### BACKGROUND TO THE INVENTION

Plants, like all living organisms, require a contribution of amino acids to achieve an optimum development. While plants are capable of synthesising amino acids, it is a complex process requiring carbon, oxygen, hydrogen, and nitrogen that has high energy expenditure. Providing these compounds from an external source has become inevitable in order to improve plant development, particularly during those stages where it is necessary to commit energy to produce other more complex compounds, such as phytoalexins and cyanogenic compounds for the defence against attacks by pathogens, vitamins for the development of plants, chromogenic compounds that are part of different pigments giving rise to colours in flowers and fruits, and plant hormones. Amino acids are incorporated into plants through the leaves or roots, the latter being the most common mechanism for providing amino acids from an external source, such as fertilisers.

The use of fertilisers has been and is a determining factor in the yield of plant crops of agricultural interest. Fertilisers and pesticides have been synthesised chemically in an inexpensive manner. Frequently used fertilisers contain mixtures of inorganic phosphorus, potassium and nitrogen (known as NPK). Other chemical fertilisers also contain amino acids components. However, the adverse effects that these chemical fertilisers have on the environment have given rise to new regulations which limit their use. This is further combined with society's growing interest in ecological agriculture. All these have triggered a search for new biological sources of amino acids.

Most amino acids used in fertilisers result from the chemical or enzymatic hydrolysis of organic (animal and/or plant) residues, or algae extracts (Schmidt et al., 2003, Golf Course Management 71:91-94). Other approaches for generating amino acids exploit the ability of microorganisms to overproduce a single amino acid. The amino acids produced are then added to fertilisers.

Some biological fertilisers exploit the make use of live plant growth promoting rhizobacteria (PGPR). PGPRs are microorganisms that naturally colonise the rhizosphere, which are capable of producing nutrients for the host plant, particularly by solubilizing phosphates and iron. Hence, PGPRs directly affect root growth and development. One such PGPRs is CECT 7415 *Pseudomonas putida* BIRD-1 strain, which has the ability to solubilise insoluble phosphate and iron under extreme low humidity conditions (Roca et al., Environ. Microbiol. 15:780-794), Some PGPRs stimulate plant growth by means of controlling pathogens (Vessey J.K., 2003, Plant and Soil 255:571-86) through the induction of systemic resistance or via the production of antibiotics (Pieterse, C.M.J., et al., 2014. Annu. Rev. Microbiol. 52:347-375). No such properties are known or can be deduced from the genome of *P. putida* CECT 7415 (Matilla, et al., J. Bacteriol., 193:1290).

It would be particularly advantageous to have microorganisms capable of producing more than one type of plant growth stimulating compound to use as biofertilisers. It has proven difficult to obtain microorganisms having these characteristics and which comply with the rigorous regulations with regards to efficacy and safety.

Therefore, there is still a need in the art to provide microorganisms with improved plant growth stimulating properties.

### SUMMARY OF THE INVENTION

Using CECT 7415 *Pseudomonas putida* BIRD-1, a previously described rhizospheric strain having capacity to solubilize phosphate and iron (Roca et al., Environ. Microbiol. 15:780-794), the present inventors have generated a number of strains that are capable of producing plant growth stimulating compounds, such as L- amino acids. Advantageously, said microorganisms should furthermore produce amino acids and/or solubilize insoluble phosphates and/or iron. As shown in the Examples, said microorganisms excel in promoting plant growth in a number of plant crops of agricultural interest.

Thus, in a first aspect, the present invention relates to a variant strain of *Pseudomonas putida* BIRD-1 with accession number CECT 7415, which variant strain produces at least one plant growth-stimulating compound and/or solubilises insoluble phosphate and/or iron.

In a second aspect, the present invention relates to a supernatant of a culture of at least one variant strain according to the first aspect of the invention.

In a third aspect, the present invention relates to a fertiliser, seed, root ball, substrate, solid support, soil or field comprising at least one variant strain according to the first aspect of the invention, or a supernatant according to claims 5 and 7.

In a fourth aspect, the present invention relates to a method for stimulating plant growth, comprising the step of applying an effective amount of at least one variant strain according to the first aspect of the invention, or a supernatant according to the second aspect of the invention, or a fertiliser, substrate, solid support or soil according to the third aspect of the invention, to the plant or, alternatively, the step of planting a seed or a root ball according to the third aspect of the invention.

In a fifth aspect, the present invention relates to a method for restoring the normal state of a plant after suffering environmental stress, comprising the step of applying an effective amount of at least one variant strain according to the first aspect of the invention, or a supernatant according to the second aspect of the invention, or a fertiliser, substrate, solid support or soil according to the third aspect of the invention, to the plant or, alternatively, the step of planting a seed or a root ball according to the third aspect of the invention.

In a sixth aspect, the present invention relates to a method for solubilising an insoluble phosphate in a solid substrate or field, comprising the step of applying at least one variant strain according to the first aspect of the invention, or a fertiliser, substrate, solid support or soil according to the third aspect of the invention, to the plant or, alternatively, the step of planting a seed or a root ball according to the third aspect of the invention on the solid substrate or field.

In a seventh aspect, the present invention relates to a method for solubilising an insoluble iron in a solid substrate or field, comprising the step of applying at least one variant strain according to the first aspect of the invention, or a fertiliser, substrate, solid support or soil according to the third aspect of the invention, to the plant or, alternatively, the step of planting a seed or a root ball according to the third aspect of the invention on the solid substrate or field.

In an eighth aspect, the present invention relates to a method for solubilising an insoluble phosphate and insoluble iron in a solid substrate or field, comprising the step of applying at least one variant strain according to the first aspect of the invention, or a fertiliser, substrate, solid support or soil according to the third aspect of the invention, to the plant or, alternatively, the step of planting a seed or a root ball according to the third aspect of the invention on the solid substrate or field.

In a ninth aspect, the present invention relates to a method for obtaining a strain with capacity to produce at least one plant growth-stimulating compound and/or to solubilise insoluble phosphate and/or iron, comprising the steps of:
(i) mutagenizing *P. putida* BIRD-1 strain with accession number CECT 7415 or the variant strain according to the first aspect of the invention in the presence of an analogue of the growth-stimulating compound and
(ii) selecting strains which are capable of growing in the presence of an analogue of the growth-stimulating compound.

In a tenth aspect, the present invention relates to a strain obtainable by the method according to ninth aspect of the invention.

In an eleventh aspect, the present invention relates to the use of the variant strain according to the first aspect of the invention, or the supernatant according to the second aspect of the invention, or the fertiliser, seed, root ball, substrate, active solid support, or soil according to the third aspect of the invention, for stimulating plant growth and/or for restoring the normal state of a plant after suffering environmental stress and/or for obtaining a strain with capacity to produce at least one plant growth-stimulating compound and/or to solubilise insoluble phosphate and/or iron.

### DETAILED DESCRIPTION OF THE INVENTION

### 1. Variant strain of the invention

In a first aspect, the present invention relates to a variant strain of *Pseudomonas putida* BIRD-1 with accession number CECT 7415, which variant produces at least one plant growth stimulating compound and/or solubilises insoluble phosphate and/or iron, hereinafter "the variant strain of the invention".

The term "strain", as used herein, refers to a genetic variant or subtype of a microorganism species, preferably a bacterial species.

The present invention contemplates a variant strain selected from *P. putida* BIRD-1 strain, deposited in the Colección Española de Cultivos Tipo (CECT) with accession number CECT 7415, or a variant of said variant strain, which variant produces at least one plant growth stimulating compound and/or solubilises insoluble phosphate and/or iron.

The present invention contemplates variant strains of *Pseudomonas putida* BIRD-1 with accession number CECT 7415, which produce at least one plant growth stimulating compound and/or solubilise insoluble phosphate and/or iron. The CECT 7415 *P. putida* strain is a strain of the *P. putida* species having phosphate and iron solubilising capacity under extreme low humidity conditions, which has been described in WO 2010/018210.

As used herein, the term "variant" or "mutant" of a strain refers to any naturally-occurring or specifically developed strain obtained from by mutation or change in the DNA of at least one gene of the reference or parent strain, i.e. of the CECT 7415 *P. putida* BIRD-1 strain, that has the capacity to produce at least one plant growth stimulating compound and/or to solubilise insoluble phosphate and/or iron of the reference strain.

The processes of producing at least one plant growth stimulating compound, solubilising phosphates and solubilising iron are of an independent genetic nature since mutants can be obtained which have one, two or all three of these abilities.

In an embodiment, the variant strain of the invention produces or has the capacity to produce at least one plant growth stimulating compound. In another embodiment, the variant strain of the invention maintains the capacity of the parent strain to produce at least one plant growth stimulating compound.

In another embodiment, the variant strain of the invention solubilises phosphate or has the capacity to solubilise phosphate. In another embodiment, the variant strain of the invention maintains the capacity of the parent strain to solubilise phosphate. In a particular embodiment, the phosphate is insoluble phosphate.

In another embodiment, the variant strain of the invention solubilises iron or has the capacity to solubilise iron. In another embodiment, the variant strain of the invention maintains the capacity of the parent strain to solubilise iron.

In an embodiment, the variant strain of the invention produces or has the capacity to produce at least one plant growth stimulating compound and solubilises phosphate or has the capacity to solubilise phosphate. In another embodiment, the variant strain of the invention maintains the capacity of the parent strain to produce at least one plant growth stimulating compound and solubilise phosphate.

In an embodiment, the variant strain of the invention produces or has the capacity to produce at least one plant growth stimulating compound and solubilises iron or has the capacity to solubilise iron. In another embodiment, the variant strain of the invention maintains the capacity of the parent strain to produce at least one plant growth-stimulating compound and solubilise iron.

In an embodiment, the variant strain of the invention produces or has the capacity to produce at least one plant growth stimulating compound, solubilises phosphate or has the capacity to solubilise phosphate, and solubilises iron or has the capacity to solubilise iron. In another embodiment, the variant strain of the invention maintains the capacity of the parent strain to produce at least one plant growth stimulating compound and solubilise phosphate and iron.

Variants may or may not have the same identifying biological characteristics of the specific strains exemplified herein, provided they share similar advantageous properties in terms of the capacity to produce at least one plant growth stimulating compound and/or to solubilise insoluble phosphate and/or iron of the reference strain. For example, the 16S rRNA genes of a "variant strain" as contemplated herein may share about 85%, 86%, 87%, 88%, 89%, 90%, 91%, 92%, 93%, 94%, 95%, 96%, 97%, 98% or 99% sequence identity with a strain disclosed herein.

In another embodiment, a variant of the strains according to the present invention refer to any strain the genome of which hybridizes under stringent conditions with the genome of the CECT 7415 *P. putida* BIRD-1 strain. In general, a low stringency hybridization reaction is carried out at about 40 degrees centigrade in 10xSSC or a solution of equivalent ionic strength/temperature. A moderate stringency hybridization is typically performed at about 50 degrees centigrade in 6xSSC, and a high stringency hybridization reaction is generally performed at about 60 degrees centigrade in 1xSSC.

In another embodiment, the degree of relatedness between the variant and the parent strains is determined as the average nucleotide identity (ANI), which detects the DNA conservation of the core genome (Konstantinidis & Tiedje, 2005, Proc Natl Acad Sci USA 102: 2567-92). In some embodiments, the ANI between the variant and the parent strain is of about 95%, about, 96%, about 97%, of about 98%, of about 99%, of about 99,1%, of about 99.5%, of about 99.6%, of about 99.7%, of about 99.8%, of about 99.9%, of about 99.99%, of about 99.999%, of about 99.9999%, of about 99.99999%, of about 99.999999% or more but less than 100%.

In another embodiment, the degree of relatedness between the variant and the parent strains is determined as the Tetranucleotide Signature Frequency Correlation Coefficient, which is based on oligonucleotide frequencies (Bohlin et al., 2008, BMC Genomics 9:104). In some embodiments, the Tetranucleotide Signature Frequency Correlation coefficient between the variant and the parent strain is of about 0.99, 0.999, 0.9999, 0.99999, 0.999999, 0.999999 or more but less than 1.

In another embodiment, the degree of relatedness between the variant and the parent strains is determined as the degree of similarity obtained when analysing the genomes of the parent and of the variant strain by Pulsed-field gel electrophoresis (PFGE) using one or more restriction endonucleases. The degree of similarity obtained by PFGE can be measured by the Dice similarity coefficient. In some embodiments, the Dice similarity coefficient between the variant and the parent strain is of about 95%, about, 96%, about 97%, of about 98%, of about 99%, of about 99.1%, of about 99.5%, of about 99.6%, of about 99.7%, of about 99.8%, of about 99.9%, of about 99.99%, of about 99.999%, of about 99.9999%, of about 99.99999%, of about 99.999999% or more but less than 100%.

In another embodiment, a strain is considered a variant of a given parent strain when both strains have the same ribotype, as obtained using any of the methods known in the art as described, for instance, by Bouchet et al. (2008, Clin Microbiol Rev 21:262-73).

In another embodiment, the degree of relatedness between the variant and the parent strains is the Pearson correlation coefficient obtained by comparing the genetic profiles of both strains obtained by repetitive extragenic palindromic element-based PCR (REP-PCR) (see e.g. Chou & Wang, 2006, Int J Food Microbiol 110:135-48; Aranda-Olmedo et al., 2002, Nucleic Acids Res 30:1826-1833; Tobes and Ramos, 2005, Environ. Microbiol.,7 :225-228). In some embodiments, the Pearson correlation coefficient obtained by comparing the REP-PCR profiles of the variant and the parent strain is of about 0.99, 0.999, 0.9999, 0.99999, 0.999999, 0.999999 or more but less than 1.

In another embodiment, the degree of relatedness between the variant and the parent strains is the linkage distance obtained by comparing the genetic profiles of both strains obtained by Multilocus sequence typing (MLST) (see e.g. Maiden M. C., 1998, Proc Natl Acad Sci USA 95:3140-5). In some embodiments, the linkage distance obtained by MLST of the variant and the parent strain is of about 0.99, 0.999, 0.9999, 0.99999, 0.999999, 0.999999 or more but less than 1.

In an embodiment, the variant of the CECT 7415 *Pseudomonas putida* BIRD-1 strain is selected from the group consisting of:
- *P. putida* BIRD-1-12 with accession number CECT 9761,
- *P. putida* BIRD-1-12F with accession number CECT 9762,
- *P. putida* BIRD-1-12S with accession number CECT 9763, and
- *P. putida* BIRD-1-21 with accession number CECT 9764,

In a particular embodiment, the variant strain of the invention is *P. putida* BIRD-1-12 and BIRD-1-21 with accession number CECT 9761 and CECT 9764, respectively. These strains solubilize phosphates and iron and, further, produce L-tryptophan (Examples 2 and 3).

In a particular embodiment, the variant strain of the invention is *P. putida* BIRD1-12F with accession number CECT 9762. This strain has the capacity to produce L-tryptophan and L-phenylalanine (Examples 5, 6 and 7).

In another particular embodiment, the variant strain of the invention is *P. putida* BIRD1-12S with accession number CECT 9763. This strain also produces L-phenylalanine (Examples 10 and 11), L-methionine and L-arginine.

The present invention also contemplates variants of the CECT 9761 *P. putida* BIRD-1-12, CECT 9762 *P. putida* BIRD-1-12F, , CECT 9763 *P. putida* BIRD-1-12S, and CECT 9764 *P. putida* BIRD-1-21, strains, which maintain the capacity of the parent strain to produce at least one plant growth stimulating compound.

The present invention also contemplates variants of the CECT 9761 *P. putida* BIRD-1-12, CECT 9762 *P. putida* BIRD-1-12F, CECT 9763 *P. putida* BIRD-1-12S, and CECT 9764 *P. putida* BIRD-1-21 strains, which maintain the capacity of the parent strain to solubilise phosphate.

The present invention also contemplates variants of the CECT 9761 *P. putida* BIRD-1-12, CECT 9762 *P. putida* BIRD-1-12F, CECT 9763 *P. putida* BIRD-1-12S, and CECT 9764 *P. putida* BIRD-1-21 strains, which maintain the capacity of the parent strain to solubilise iron.

The present invention also contemplates variants of the CECT 9761 *P. putida* BIRD-1-12, CECT 9762 *P. putida* BIRD-1-12F, CECT 9763 *P. putida* BIRD-1-12S, and CECT 9764 *P. putida* BIRD-1-21 strains, which maintain the capacity of the parent strain to produce at least one plant growth stimulating compound and solubilise phosphate.

The present invention also contemplates variants of the CECT 9761 *P. putida* BIRD-1-12, CECT 9762 *P. putida* BIRD-1-12F, CECT 9763 *P. putida* BIRD-1-12S, and CECT 9764 *P. putida* BIRD-1-21 strains, which maintain the capacity of the parent strain to produce at least one plant growth stimulating compound and solubilise iron.

The present invention also contemplates variants of the CECT 9761 *P. putida* BIRD-1-12, CECT 9762 *P. putida* BIRD-1-12F, CECT 9763 *P. putida* BIRD-1-12S, and CECT 9764 *P. putida* BIRD-1-21 strains which maintain the capacity to produce at least one plant growth stimulating compound and to solubilise insoluble phosphate and iron.

Variants according to the present invention include those variants maintaining at least 40%, at least 50%, at least 60%, at least 70%, at least 80%, at least 90%, at least 95%, at least 96%, at least 97%, at least 98%, at least 99%, at least 100%, at least 110%, at least 120%, at least 130%, at least 140%, at least 150% or more or the capacity to produce at least one plant growth stimulating compound with respect to the capacity to produce at least one plant growth stimulating compound of the parent strain.

Variants according to the present invention include those variants maintaining at least 40%, at least 50%, at least 60%, at least 70%, at least 80%, at least 90%, at least 95%, at least 96%, at least 97%, at least 98%, at least 99%, at least 100%, at least 110%, at least 120%, at least 130%, at least 140%, at least 150% or more of the capacity to solubilise insoluble phosphate with respect to the capacity to solubilise phosphate of the parent strain.

Variants according to the present invention include those variants maintaining at least 40%, at least 50%, at least 60%, at least 70%, at least 80%, at least 90%, at least 95%, at least 96%, at least 97%, at least 98%, at least 99%, at least 100%, at least 110%, at least 120%, at least 130%, at least 140%, at least 150% or more capacity to solubilise iron with respect to the capacity to solubilise iron of the parent strain.

The term "capacity to produce at least one plant growth stimulating compound", as used herein, refers to the property by which a strain is able to synthesise and release to the soil at least one plant growth stimulating compound. The term "plant growth stimulating compound" or "plant growth promoting compound", as used herein, refers to compounds which promote plant growth by facilitating the development of the plant root system, stem elongation, flowering, etc. Examples of plant growth stimulating compounds that may be produced by the variant strain of the invention includes, without limitation, nitrogen sources, such as amino acids; phytohormones, such as indole 3-acetic acid (IAA), gibberellic acid (GA3), ethylene, zeatin, and abscisic acid (ABA); and plant growth regulatory compounds, such as polyamines, e.g. cadaverine (CAD).

If the variant strain of the invention is administered to a plant, its ability to produce an amino acid enables the plant to prevent the energy expenditure involved in synthesising the same amino acid from inorganic nitrogenous compounds of the soil, thereby having that energy available for other purposes. Furthermore, certain amino acids have additional functions. For example, L-tryptophan is a precursor of indoleacetic acid (IAA), which is a compound that promotes root development; and L-phenylalanine is a compound which promotes seed germination. Additionally, the absorption of amino acids by the plant provides the plant with greater resistance to abiotic stresses such as, for example, adverse weather conditions, changes in temperature, and desiccation or water stress conditions. (Manela et al., 2015, Front. Plant Sci 6:538)

In an embodiment, the at least one plant growth stimulating compound is an L- amino acid. The at least one L- amino acid may be any one of the 21 amino acids found in eukaryotes, or combinations thereof.

In a particular embodiment, the L- amino acid is selected from the group consisting of L-Tryptophan, L-Phenylalanine, L-Arginine, L-methionine preferably, L-Tryptophan.

In a preferred embodiment, the L-amino acid is L-Tryptophan and the strain further produces at least one L- amino acid. The at least one further L- amino acid can be any one of the 20 remaining amino acids, or combinations thereof. Preferably, the at least one further L- amino acid is selected from the group consisting of L-Phenylalanine, L-Methionine and L-Arginine

The ability of a microorganism to produce plant growth stimulating or promoting compounds can be determined by means of any conventional method, for example, by means of an early plant growth stimulation assay. Briefly, said assay comprises sowing seeds, e.g. corn, barley, grass, or Avex III (herbage mixture), in a homogenous mixture made up of agricultural soil mixed with sand (silica) and a culture of the microorganism to be assayed (in a suitable population density), preferably adhered to a solid support. The system is incubated under conditions that allow seed germination (e.g. at 20 °C in the dark). The percentage of seed germination and/or the length of the stem is determined a week after sowing [Examples 18 to 36].

The ability of a microorganism to produce each type of plant growth stimulating compound may also be determined separately. Production of phytohormones may be determined by suitable chromatography techniques, such as reverse phase chromatography and gas chromatography-mass spectrometry with selective ion monitoring (GC-MS-SIM), as described by Perrig et al., 2007, (Appl. Microbiol. Biotechnol. 75:1143-50). Ethylene production may be determined by, for example, gas chromatography-flame ionisation detection. The production of CAD and other polyamines may be evaluated, for example, by the method described in Tiburcio (1997, Physiol. Plant 100:664-74). One skilled in the art will understand that other assays that serve to evaluate the plant growth stimulation or promotion can be conducted, such as, for example, the assays mentioned in international patent application WO 2011/147826, which is incorporated herein in its entirety by reference.

The ability of a strain to produce a given amino acid can be determined by any conventional method, for example, by inoculating a culture of said strain in a medium containing an analogue of the amino acid at hand, for example, 5-fluoro-D,L-tryptophan, p-fluoro-D,L-phenylalanine, sulfoguanidine, norvaline, methionine-D,L-sulfoximine, etc., and incubating it under suitable conditions, such as those described in Examples 2 to 14, and then comparing the amount of amino acid produced by the strain with the amount of amino acid produced by a control strain, e.g. CECT 7415 *P. putida* BIRD-1 strain. The production of amino acids can be determined by conventional methods, for example, by means of conventional colorimetric methods, such as spectrophotometric methods with ferric chloride, dinitrofluorobenzene, phenylisothiocyanate, fluorescamine, ninhydrin, etc. Likewise, the amino acids can be determined, once derivatized, after separation by high-pressure liquid chromatography (HPLC). Another way to verify the production of amino acids consists in conducting cross-nutrition assays in which the microorganism to be assayed as a producer of an amino acid is inoculated in a minimal medium with the nutrients and the conditions necessary for growth. Once a culture of said microorganism has been obtained, the medium is filtered under sterile conditions with filters having a pore size of 0.22 µm to remove the cells and is re-inoculated with an auxotrophic microorganism for the corresponding amino acid. Under these conditions, the increase in bacterial density of the auxotrophic microorganism is indicative of the presence of amino acid (Riccardi et al., Production of amino acids by analogue-resistant mutants of the Cyanobacterium Spirulina platensis (1981) Journal of Bacteriology 147:1002). Examples 2-14 included in the present description illustrate obtaining and isolating strains of *P. putida* that produce different amino acids (L-tryptophan, L-phenylalanine, L-methionine, L-arginine ), where said strains can be variants of *P. putida* BIRD-1-12 (CECT 9761) or of the *P. putida* parental strain BIRD-1 (CECT 7415), which produce amino acids.

The term "capacity to solubilise phosphate", as used herein, refers to the ability of a strain that is able to release and solubilise phosphate from an insoluble phosphate source, such as the phosphate present, for example, in soils or in rock phosphate [e.g. dibasic calcium phosphate (CaHPO₄ x 2H₂O), tribasic calcium phosphate (Ca₅ (PO₄)₃OH or TPC), etc.], and using it as a phosphorus source. Phosphate solubilisation by a microorganism may be assessed by methods that are conventional in the art. For example, by inoculating a culture of said microorganism in a medium containing one or more insoluble phosphates as the sole phosphorus source (e.g., tricalcium phosphate, etc.), incubating under suitable conditions and counting the viable microorganisms, as described in Examples 1, 15 and 16 under these conditions, the maintenance or an increase of the bacterial population density is indicative that said microorganisms solubilize said insoluble phosphate/phosphates and use it/them as a phosphorus source (Rodriguez & Fraga, 1999, Biotechnology Advances 17:319-39). Another assay which allows clarifying if a microorganism is capable of solubilising insoluble phosphates comprises seeding them in solid media specialized for this purpose, in which the microorganism generates a microenvironment due to the production of organic acids, which translates into a visible halo in the medium (Nautiyal, C.S., 1999 Microbiology Letters 170:265-70).

The term "capacity to solubilise iron", as used herein, refers to the ability of a strain to solubilise insoluble iron present, for example, in soils or in rock phosphate (e.g. iron oxides, etc.). The most common way for the microorganisms to solubilize iron is by means of producing, which are small, high affinity iron chelating compounds that are re-assimilated by the strain of the invention and facilitate iron uptake by plants. Siderophore production may be determined by any conventional method, for example, by inoculating pure bacterial culture in plates containing CAS agar and observing for orange colour formation around each colony upon incubation. The ability of a microorganism to solubilise iron can also be determined by inoculating a culture of said microorganism in a medium containing insoluble iron as the only iron source (e.g., ferric trichloride, etc.), and counting the viable microorganisms, as described in WO 2010/018210. Under these conditions, the maintenance of or an increase in the bacterial population density is indicative that said microorganisms solubilise said insoluble iron(s) and use it/them as an iron source. Another example consists in inoculating a culture of said microorganisms in a suitable culture medium without adding iron, incubating under suitable conditions and counting the viable microorganisms, as described in Example 1; under these conditions, the maintenance or an increase of the bacterial population density is indicative that said microorganisms use iron, or, the formation of a greenish-yellow color in the supernatants of the cultures is indicative of the excretion of certain siderophores into the culture medium.

The CECT 7415 *P. putida* BIRD-1 strain produces at least one plant growth stimulating compound, solubilises insoluble phosphate and iron, as demonstrated in Example 1 of the present application.

It will be recognised that the supernatant of the invention contains the products derived from the metabolism of the variant strain of the invention, i.e. at least one plant growth stimulating compound and/or any element which helps solubilising insoluble phosphate and/or iron. Advantageously, the supernatant may contain an amino acid, preferably an L-amino acid, such as an amino acid selected from the group consisting of L-tryptophan, L-phenylalanine, L-methionine and L-arginine or any combination thereof. These features allow the supernatant of the invention to have multiple applications in the field of agriculture, such as use as a fertiliser and plant strengthener, for example. Therefore, the invention also contemplates the supernatant of a culture of at least one variant strain according to the invention, hereinafter "the supernatant of the invention".

As used herein, the term "supernatant" refers to a cell-free culture medium which has been conditioned by the growth of a microorganism and which, as a result of the conditioning, contains compounds that have been synthesised and secreted by the microorganism. In order to obtain a supernatant according to the invention, a culture medium is inoculated with the variant strain of the invention, which is cultured under suitable conditions. For example, the variant strain of the invention may be cultured in a minimal culture medium comprising a carbon source that can be used by the strain. Said carbon source may comprise, without limitation, glucose, sucrose, lactic acid , benzoic acid, citric acid, or combinations thereof. Virtually any culture medium suitable for the development and growth of *P. putida* can be used in putting said method into practice. Additionally, the culture medium may comprise other media specific for achieving production of the desired metabolite. Said culture media are well known in the state of the art and preparing them is routine practice for one skilled in the art. The strain may be cultured at a temperature between 15 °C and 40 °C until a cell density equal to or greater than 10⁹ cfu/mL is reached. The cells are then removed from the medium by any suitable means, such as decantation, centrifugation or filtration. Examples 1 and 2 describe conditions and culture media suitable for the development and growth of *P. putida* BIRD-1 and the mutants thereof.

Where the culture broth or supernatant is not going to be used immediately, it can be preserved until use under cold conditions, e.g. at 4°C, -20 °C, or -80 °C.

A biologically pure culture of a variant strain of the invention is an additional aspect of the invention.

### 2. Products containing the variant strain of the invention

### 2.1. Fertiliser

In another aspect, the invention relates to a fertiliser comprising at least one variant strain of the invention, hereinafter "the first fertiliser of the invention".

The terms "variant strain of the invention" and "supernatant of the invention" have been described previously in detail and their definitions and embodiments are included here by reference.

The term "fertiliser", as used herein, refers to any compound which is added to a soil to facilitate plant growth. The strain of the invention can be added to virtually any fertiliser, either solid or liquid, for putting the present invention into practice. Non-limiting illustrative examples of fertilisers are: single nutrient fertilisers, binary fertilisers, and NPK-type fertilisers. The main nitrogen-based single nutrient fertiliser is ammonia or its solutions. Ammonium nitrate and urea are also widely used. The main straight phosphate fertilisers are the superphosphates, which comprise varying amounts of various phosphates, such as P₂O₅. Binary fertilisers, or NP, or NK or PK fertilisers, provide two-component combinations of nitrogen, phosphorus and potassium. NPK fertilisers are three-component fertilisers providing nitrogen, phosphorus, and potassium.

Any liquid fertiliser with a pH comprised between 2 and 10 may also be used for putting this invention into practice. Non-limiting examples of liquid fertilisers include fertilisers based on fulvic acids, liquid fertilisers with different compositions, such as 16:2:4 mixtures, 20:5:0 mixtures, etc., or various types of fertilisers in drops with an NPK composition of 8:9:9, 16:4:4, etc. The variant strain of the invention can be added to the fertiliser.

The fertiliser herein described may contain, if desired, other ingredients or constituents commonly used in agricultural compositions, such as solvents, active agents, or pH regulators, provided that they do not jeopardize or compromise the viability of the variant strain of the invention. Said ingredients or constituents commonly used in agricultural compositions are generally known to those skilled in the art. The main micronutrients widely used are molybdenum, zinc, and copper although the micronutrient needs depend on the plant. For example, sugar beets appear to require boron, and legumes require cobalt.

The fertiliser of the invention can be obtained by conventional methods by mixing said fertiliser with a culture containing one or more of the strains of the invention. Said mixture is made with the suitable weight:volume (w:v) proportion which is greatly variable depending on, among other factors, the colony forming units (cfu) present in the culture and the cfu per unit of measure (g or mL) of fertiliser to be obtained in the supplemented fertiliser of the invention.

In a particular embodiment, between 0.01 and 1 mL of culture containing 10⁷ cfu of the variant strain of the invention are mixed with 1 g of solid fertiliser. In another specific embodiment, 1 mL of culture containing 10⁷ cfu of the variant strain of the invention is mixed with 1 mL of liquid fertiliser with a pH comprised between 2 and 10. It will be understood that, when more than one variant strain of the invention is added to the fertiliser, the previous ratios are calculated using the combined cultures.

In order to facilitate adhesion of the variant strain of the invention to the fertiliser, a suitable adhesive may be used. Virtually any agriculturally acceptable adhesive may be used. Non-limiting examples of said adhesives include acacia gum and polymers based on alginate, such as calcium alginate. The amount of adhesive may vary but it is typically equal to or less than 1% by weight of the total weight of the fertiliser.

The fertiliser of the invention may be obtained by mixing a fertiliser with a pure culture containing the variant strain of the invention, or with several pure cultures each containing a different variant strain of the invention. Conveniently, the fertiliser adsorbs at least 10³ cfu/g of fertiliser, at least 10⁴ cfu/g of fertiliser, at least 10⁵ cfu per g of fertiliser, at least 10⁶ cfu/g of fertiliser, at least 10⁷ cfu/g of fertiliser, at least 10⁸ cfu/g of fertiliser, at least 10⁹ cfu/g of fertiliser, at least 10¹⁰ cfu/g of fertiliser, or more.

The mixture of the fertiliser with the culture containing the variant strain of the invention can generally be made based on a weight:volume (w:v) proportion that varies greatly, depending, among other factors, on the cfu/mL present in the culture and on the cfu per gram of fertiliser to be obtained in the fertiliser of the invention.

The fertiliser of the invention may comprise 2, 3, 4, or up to 5 different strains of the invention. Additionally, the fertiliser of the invention may comprise 2, 3, 4, or up to 5 different strains of the invention together with one or more strains available in the art which show one or more properties adequate for being used as fertilizers, i.e. capacity to produce one or more plant growth-stimulating compounds and/or capacity to solubilize insoluble phosphate and/or iron.

The presence of the variant strain of the invention in the fertiliser provides that the fertiliser would contribute not only to reducing the environmental problem relating to the accumulation of insoluble phosphate on arable soils since it would enable its assimilation, but it would also contribute to reducing pathogen growth on the treated surface since it would remove iron, a necessary element for pathogens; from the surrounding area. Solid and liquid fertilisers thus supplied would play a dual role in the development of plants, namely (i) facilitating nutrients and stimulating growth and (ii) preventing pathogen growth (e.g., fungi, bacteria, etc.) for plants.

It will be understood that the supernatant of the invention also provides beneficial compounds to a fertiliser. Thus, in another aspect, the invention relates to a fertiliser comprising at least one supernatant of the invention, hereinafter "the second fertiliser of the invention".

The particular features of the first fertiliser of the invention may be conveniently adapted for the second fertiliser of the invention.

### 2.2. Substrate

As one skilled in the art will understand, the application of the variant strain of the invention to plants may be facilitated by fixing the strain on a substrate. The substrate will advantageously favour the proliferation of said strain in the plant rhizosphere. Therefore, in another aspect, the invention relates to a substrate comprising at least one variant strain of the invention, hereinafter "the first substrate of the invention".

The terms "variant strain of the invention" and "supernatant of the invention" have been described previously in detail and their definitions and embodiments are included here by reference.

The term "substrate", as used herein, is an inert soilless substrate that is composed mainly of organic elements and that is able to sustain plants. Virtually any substrate that meets said conditions may be used in the present invention. Non-limiting examples of substrates include peat (peat moss), bark, coir dust, cork dust; cellulose, all of which were previously dried, and ground algae extracts, compost, and mixtures thereof.

The substrate of the invention may be obtained by mixing said substrate with a pure culture containing the variant strain of the invention or with more than one pure culture each containing a different variant strain of the invention. Conveniently, the substrate adsorbs at least 10³ cfu/g of substrate, at least 10⁴ cfu/g of substrate, at least 10⁵ cfu per g of substrate, at least 10⁶ cfu/g of substrate, at least 10⁷ cfu/g of substrate, at least 10⁸ cfu/g of substrate, at least 10⁹ cfu/g of substrate, at least 10¹⁰ cfu/g of substrate, or more.

The mixture of the substrate with the culture containing the variant strain of the invention can generally be made based on a weight:volume (w:v) proportion that varies greatly, depending, among other factors, on the cfu/mL present in the culture and on the cfu per gram of substrate to be obtained in the substrate of the invention. Nevertheless in a particular embodiment, said ratio substrate :culture containing the variant strain of the invention is comprised between 1:0.01 and 1:1 (w:v); in a specific embodiment, 1 ml of culture containing 10⁷ of the variant strain of the invention is mixed with 1 gram of substrate.

The substrate of the invention may comprise 2, 3, 4 or up to 5 different strains of the invention.

It will be understood that the supernatant of the invention also provides beneficial compounds to a substrate. Thus, in another aspect, the invention relates to a substrate comprising at least one supernatant of the invention, hereinafter "the second substrate of the invention".

The particular features of the first substrate of the invention may be conveniently adapted for the second substrate of the invention.

### 2.3. Active solid support

As one skilled in the art will understand, the application of the variant strain of the invention to plants may be facilitated by fixing the strain on a solid support. The solid support will advantageously favour the proliferation of said strain in the plant rhizosphere. Therefore, in another aspect, the invention relates to a solid support comprising at least one variant strain of the invention, hereinafter "the first active solid support of the invention".

The terms "variant strain of the invention" and "supernatant of the invention" have been described previously in detail and their definitions and embodiments are included here by reference.

The term "solid support" or "active solid support", as used herein, is an inert solid support which has a high surface area to enable a high adsorption capacity of microorganisms (Busscher & Weerkamp, 1999, FEMS Microbiology Letters 46:465). Virtually any solid support that meets said conditions may be used in the present invention. Non-limiting examples of solid supports include clay, such as bentonite and sepiolite, perlite, styrofoam, vermiculite, talcum, rockwool, and mixtures thereof.

The active solid support of the invention may be obtained by mixing said solid support with a pure culture containing the variant strain of the invention, or with more than one pure culture each containing a different variant strain of the invention. Conveniently, the solid support adsorbs at least 10³ cfu/g of solid support, at least 10⁴ cfu/g of solid support, at least 10⁵ cfu per g of solid support, at least 10⁶ cfu/g of solid support, at least 10⁷ cfu/g of solid support, at least 10⁸ cfu/g of solid support, at least 10⁹ cfu/g of solid support, at least 10¹⁰ cfu/g of solid support, or more.

The mixture of the active solid support with the culture containing the variant strain of the invention can generally be made based on a weight:volume (w:v) proportion that varies greatly, depending, among other factors, on the cfu/mL present in the culture and on the cfu per gram of solid support to be obtained in the active solid support of the invention. Nevertheless in a particular embodiment, said ratio solid support:culture containing the variant strain of the invention is comprised between 1:0.01 and 1:1 (w:v); in a specific embodiment, 1 ml of culture containing 10⁷ of the variant strain of the invention is mixed with 1 gram of solid support.

The active solid support of the invention may comprise 2, 3, 4 or up to 5 different strains of the invention.

It will be understood that the supernatant of the invention also provides beneficial compounds to a solid support. Thus, in another aspect, the invention relates to a solid support comprising at least one supernatant of the invention, hereinafter "the second active solid support of the invention".

The particular features of the first active solid support of the invention may be conveniently adapted for the second active solid support of the invention.

### 2.4. Seed

In another aspect, the present invention relates to a seed comprising at least one variant strain of the invention, hereinafter "the seed of the invention".

The terms "variant strain of the invention" and "supernatant of the invention" have been described previously in detail and their definitions and embodiments are included here by reference.

The term "seed", as used herein, refers to an embryonic plant enclosed in a protective outer covering, which is formed as part of the process of reproduction in plants. The seed may be the seed of any plant with agricultural, forestry, food (for human or animal purposes), ornamental, or energy interest, etc.

The variant strain of the invention may be present partially or completely on the seed surface.

The seed of the invention may be obtained by conventional methods. By way of illustration, it can be obtained by submerging the seed in a suspension or pure culture of the variant strain of the invention or, alternatively, by spraying said suspension or culture on the seeds. The suspension or pure culture may contain more than one variant strain of the invention. Other methods include mixing the seed and the variant strain of the invention previously incorporated in a solid support to facilitate adhesion of the variant strain of the invention to said seed.

The seed of the invention may comprise at least 10³ cfu/g of seed, at least 10⁴ cfu/g of seed, at least 10⁵ cfu per g of seed, at least 10⁶ cfu/g of seed, at least 10⁷ cfu/g of seed, at least 10⁸ cfu/g of seed, at least 10⁹ cfu/g of seed, at least 10¹⁰ cfu/g of seed, or more.

Furthermore, in order to improve adhesion of the variant strain of the invention to the seed, an agricultural type adhesive containing a suitable concentration of said strain, such as an organic type gel or polymer, such as acacia gum or alginate polymers, all of which are inert for the variant strain of the invention, may be used.

The seed of the invention may comprise 2, 3, 4 or up to 5 different strains of the invention.

It will be understood that the supernatant of the invention also provides beneficial compounds to a seed. Thus, in another aspect, the invention relates to a seed comprising at least one supernatant of the invention, hereinafter "the second seed of the invention".

The particular features of the first seed of the invention may be conveniently adapted for the second seed of the invention.

### 2.5. Root ball

In another aspect, the present invention relates to a root ball comprising at least one variant strain of the invention, hereinafter "the first root ball of the invention".

The terms "variant strain of the invention" and "supernatant of the invention" have been described previously in detail and their definitions and embodiments are included here by reference.

As used herein, the term "root ball" refers to the main mass of roots at the base of a plant. The root ball is of particular importance when a plant is re-potted or planted out in the ground since the quality and preparation of the root ball will determine how well the plant will survive this transplantation. The root ball may be the root ball of with agricultural, forestry, food (for human or animal purposes), ornamental, or energy interest, etc. At least one variant strain of the invention may completely or partially coat the root ball.

The root ball of the invention may be obtained by conventional methods. By way of illustration, it can be obtained by submerging the seed in a suspension or pure culture of the variant strain of the invention or, alternatively, by spraying said suspension or culture on the root ball. The suspension or pure culture may contain more than one variant strain of the invention. Other methods include mixing the root ball and the variant strain of the invention previously incorporated in a solid support to facilitate adhesion of the variant strain of the invention to said root ball.

The root ball of the invention may comprise at least 10³ cfu/g of root ball, at least 10⁴ cfu/g of root ball, at least 10⁵ cfu per g of root ball, at least 10⁶ cfu/g of root ball, at least 10⁷ cfu/g of root ball, at least 10⁸ cfu/g of root ball, at least 10⁹ cfu/g of root ball, at least 10¹⁰ cfu/g of root ball, or more.

Furthermore, to improve adhesion of the at least one variant strain of the invention to the root ball, an agricultural type adhesive containing a suitable concentration of said strain, such as an organic type gel or polymer, such as acacia gum or alginate polymers, all of which are inert for the variant strain of the invention, may be used.

The root ball of the invention may comprise 2, 3, 4 or up to 5 different strains of the invention.

It will be understood that the supernatant of the invention also provides beneficial compounds to a root ball. Thus, in another aspect, the invention relates to a root ball comprising at least one supernatant of the invention, hereinafter "the second root ball of the invention".

The particular features of the first root ball of the invention may be conveniently adapted for the second root ball of the invention.

### 2.6. Soil

In another aspect, the present invention relates to a soil comprising at least one variant strain of the invention, hereinafter "the soil of the invention".

The soil of the invention may comprise 2, 3, 4 or up to 5 different strains of the invention.

The invention contemplates any soil that comprises the strain(s) of the invention. Accordingly, the variant strain of the invention may be provided to the soil either directly or by means of the fertiliser of the invention, the active solid support of the invention, the seed of the invention or the root ball of the invention.

Any soil which is capable of sustain plants may be used.

It will be understood that the supernatant of the invention also provides beneficial compounds to a soil. Thus, the invention also relates to a soil comprising at least one supernatant of the invention.

The terms "variant strain of the invention", "supernatant of the invention", "fertiliser of the invention", "substrate of the invention", "active solid support of the invention", "soil of the invention", "seed of the invention", and "root ball of the invention", have been described previously in detail and their definitions and embodiments are included here by reference.

### 2.7. Field

In another aspect, the present invention relates to a field comprising at least one variant strain of the invention, hereinafter "the field of the invention".

The field of the invention may comprise 2, 3, 4, or up to 5 strains of the invention.

The invention contemplates any field that comprises the strain(s) of the invention. Accordingly, the variant strain of the invention may be provided to the field either directly or by means of the fertiliser of the invention, the active solid support of the invention, the seed of the invention, the root ball of the invention or the soil of the invention.

Any field which is capable of sustain plants may be used.

It will be understood that the supernatant of the invention also provides beneficial compounds to a field. Thus, the invention also relates to a field comprising at least one supernatant of the invention.

The terms "variant strain of the invention", "supernatant of the invention", "fertiliser of the invention", "substrate of the invention", "active solid support of the invention", "soil of the invention", "seed of the invention", and "root ball of the invention", have been described previously in detail and their definitions and embodiments are included here by reference.

The field may have a surface of at least 0.1 m², or at least 1 m², or at least 10 m², or at least 100 m², or at least 1,000 m², or at least 10,000 m², or at least 100,000 m², or more.

### 3. Methods of use

It will be readily appreciated that the variant strain of the invention has multitude of applications in agriculture. The methods to exploit the use of the variant strains of the invention are also part of the present invention.

### 3.1. Method for stimulating plant growth

The presence of the variant strain of the invention or the supernatant of the invention is particularly advantageous in agriculture because they promote the simultaneous germination of the seeds, provide for homogenous growth of the plants of the same crop, and coordinate the flowering, fruiting, and fruit maturation periods.

In another aspect, the invention relates to a method for stimulating plant growth, hereinafter "the method for stimulating plant growth of the invention", comprising the step of applying an effective amount of at least one variant strain of the invention.

The capacity of the variant strain of the invention to produce at least one plant growth stimulating compound and/or to solubilise insoluble phosphate and/or iron is particularly advantageous.

In an embodiment, an effective amount of at least two, at least three, at least 4, or at least 5, different strains of the invention are applied to the plant.

Alternatively, the method for stimulating plant growth of the invention may comprise the step of applying the supernatant of the invention to the plant.

It will be readily appreciated that the variant strain of the invention can be applied in different ways, i.e. directly to the soil, preferably to the radicular area of influence of the plant, or as part of a fertiliser, adhered to a solid support, or as part of a soil.

Alternatively, the method for stimulating plant growth of the invention may comprise the step of applying the fertiliser of the invention to the plant.

Alternatively, the method for stimulating plant growth of the invention may comprise the step of applying the active solid support of the invention to the plant.

Alternatively, the method for stimulating plant growth of the invention may comprise the step of applying the soil of the invention to the plant.

Alternatively, the growth of a plant may be stimulated by means of sowing a seed or a root ball of said plant supplemented with the variant strain of the invention, i.e. the seed or root ball of the invention. In this case, the variant strain of the invention will replicate and populate the area of soil surrounding the seed or root, and exert its function in stimulating plant growth.

Alternatively, the method for stimulating plant growth of the invention may comprise the step of sowing the seed of the invention or the root ball of the invention near the plant whose growth is to be stimulated. Provided that the seed or root ball are planted sufficiently near the radicular area of influence of the plant whose, the variant strain of the invention will replicate and populate nearby areas of the rhizosphere.

The terms "variant strain of the invention", "supernatant of the invention", "fertiliser of the invention", "substrate of the invention", "active solid support of the invention", "soil of the invention", "seed of the invention", and "root ball of the invention", have been described previously in detail and their definitions and embodiments are included here by reference.

The term "effective amount", as used in the context of this aspect of the invention, refers to the amount of the variant strain of the invention, the supernatant of the invention, or the products containing said strain or supernatant according to the invention, that is required to stimulate, enhance or induce plant growth, i.e. to increase the biomass of said plant. The term "plant biomass", as used herein, refers to the amount of organic material contained in a plant, i.e., the organic material constituting both the aerial part of the plant, that is, the stem, the trunk, the leaves, the branches, the fruit, the inflorescences, etc. (aerial biomass), and the underground part thereof, i.e., the roots, calluses, tubercles, etc. (underground biomass). The "plant biomass" is often measured as the dry mass or weight (or "fresh weight," where appropriate) of the plant. As known the skilled person, there are multiple methods and parameters serving to calculate plant growth or the increase in biomass in the state of the art, including, without limitation, the growth rate, the relative growth rate, the leaf area ratio, the specific leaf area, the leaf proportion, the net assimilation rate, the stem proportion, the root proportion, and the dry matter content.

The effective amount of a strain according to this aspect of the invention may be determined by methods that are conventional in the art. For example, different amounts of the variant strain of the invention, the supernatant of the invention, or the products containing said strain or supernatant according to the invention, may be applied to different plants which are at the same stage of development. Plant growth, i.e. the increase in biomass, is monitored over time to determine which of the tested amounts is effective. The increase in plant biomass may be measured as a % of biomass increase when compared with a plant that has not been treated with the variant strain of the invention.

Other indicators of plant growth stimulation may include increased stem length, increased root length, increased number of secondary roots, earlier germination of seeds, number of germinating seeds, earlier flowering, and earlier harvests.

The effective amount will depend on the particular plant, growth stage and the radicular area of influence, which may be translated as the size of the plant. The effective amount may be measured in total cfu, cfu per gram of rhizospheric soil or any other convenient unit). The effective amount of the variant strain of the invention may be between 10³ and 10⁹ cfu per gram of rhizospheric soil. The effective amount of the supernatant of the invention may be between 1 mL and 10 L per m² of soil surface. The effective amount of the fertiliser, substrate, solid support, or soil of the invention may an amount which results in between 10³ and 10⁹ cfu per gram of rhizospheric soil. Said amounts may be applied in a single administration or in several administrations.

The growth of virtually any plant may be stimulated according to this method. By way of illustration, any plant having agricultural interest may be used for this method, including plants of interest in human or animal food, plants of ornamental interest, plants of forestry interest, plants of energy interest, and so on. Non-limiting examples include oat, barley, corn, wheat, strawberry, black berries, tomato, pepper, cucumber, eggplant, grass, sorghum, rose bushes, geraniums, daisies, soya, any tree, such as fruit trees, oak tree, walnut tree, beech tree, pine tree, etc.

The method for stimulating growth of the invention may be particularly effective in the early stages of growth.

Moreover, the method for stimulating growth of the invention can be applied to both monocotyledons and dicotyledons.

There are various mechanisms by which the variant strains of the invention may stimulate plant growth. First, by producing and releasing into the soil amino acids, these enable the plant to prevent the energy expenditure involved in synthesising the same amino acid from inorganic nitrogenous compounds of the soil, thereby having that energy available for other purposes. Second, certain amino acids have additional functions. For example, L-tryptophan is a precursor of indoleacetic acid (IAA), which is a compound that promotes root development; and L-phenylalanine is a compound which promotes seed germination. Phenylalanine also promotes closing of stoma which helps to fight water stress (Tyerman, et al., 2002, Plant Cell and Environ. 25:173-194). Phenylalanine is the substrate of phenylalanine ammonia lyase that favours the synthesis of salicylic acid and hence induced systemic resistance (Mauch-Mani and Slusarenko, 1996, The Plant Cell 8:203-212): Third, the variant strains of the invention have been shown to form biofilms on the surface of roots of horticultural plants which has a protective effect against pathogenic organisms and, in turn, promotes plant growth.

### 3.2. Method for restoring the normal state of a plant after suffering environmental stress

In another aspect, the invention relates to a method for restoring the normal state of a plant after suffering environmental stress, hereinafter "the method for restoring the normal state of a plant" of the invention, comprising the step of applying an effective amount of at least one variant strain of the invention.

In an embodiment, an effective amount of at least two, at least three, at least 4, or at least 5 different strains of the invention are applied to the plant.

Alternatively, the method for stimulating plant growth of the invention comprises the step of applying the supernatant of the invention to the plant.

It will be readily appreciated that the variant strain of the invention can be applied in different ways, i.e. directly to the soil, preferably to the radicular area of influence of the plant, or as part of a fertiliser, adhered to a solid support, or as part of a soil.

Alternatively, the method for restoring the normal state of a plant of the invention may comprise the step of applying the fertiliser of the invention to the plant.

Alternatively, the method for restoring the normal state of a plant of the invention may comprise the step of applying the active solid support of the invention to the plant.

Alternatively, the method for restoring the normal state of a plant of the invention may comprise the step of applying the soil of the invention to the plant.

Alternatively, the normal state of a plant may be restored after suffering environmental stress by means of sowing the seed of the invention or the root ball of the invention near the plant whose growth is to be stimulated. Provided that the seed or root ball are planted sufficiently near the radicular area of influence of the plant whose, the variant strain of the invention will replicate and populate nearby areas of the rhizosphere.

The terms "variant strain of the invention", "supernatant of the invention", "fertiliser of the invention", "substrate of the invention", "active solid support of the invention", "soil of the invention", "seed of the invention", and "root ball of the invention", have been described previously in detail and their definitions and embodiments are included here by reference.

The term "normal state", as used herein, refers to the general or vegetative state of a plant under conditions that are optimal for plant growth.

The term "environmental stress", as used herein, refers to abiotic situations which affect the conditions that are optimal for plant growth. Non-limiting examples of environmental stress include adverse weather conditions, such as intense cold or frosts, changes in temperature, including high temperatures or thermal shock, desiccation or water stress conditions, salinity, etc.

The term "effective amount", as used in the context of this aspect of the invention, refers to the amount of the variant strain of the invention, regardless of the form of presentation, that is required to obtain the desired effect of re-establishing the normal state of a plant after an environmental stress situation and thereby obtaining beneficial results.

The effective amount of a strain according to this aspect of the invention may be determined empirically by methods that are conventional in the art. For example, different amounts of the variant strain of the invention may be applied to different plants which are at the same stage of development and have suffered the same type of environmental stress. Plant growth, i.e. the increase in biomass, is monitored over time to determine which of the tested amounts is effective.

The effective amount will depend on the particular plant, growth stage and the radicular area of influence, which may be translated as the size of the plant, and also on the particular type of environmental stress suffered by the plant. The effective amount may be measured in total cfu, cfu per gram of rhizospheric soil or any other convenient unit. The effective amount of the variant strain of the invention may be between 10³ and 10⁹ cfu per gram of rhizospheric soil. The effective amount of the supernatant of the invention may be between 1 mL and 10 L per m² of soil surface. The effective amount of the fertiliser, substrate, solid support, or soil of the invention may an amount which results in between 10³ and 10⁹ cfu per gram of rhizospheric soil. Said amounts may be applied in a single administration or in several administrations.

The normal state of virtually any plant may be restored according to this method. By way of illustration, any plant having agricultural interest may be used for this method, including plants of interest in human or animal food, plants of ornamental interest, plants of forestry interest, plants of energy interest, and so on. Non-limiting examples include oat, barley, corn, wheat, strawberry, black berries, tomato, pepper, cucumber, eggplant, grass, sorghum, rose bushes, geraniums, daisies, soya, any tree, such as fruit trees, oak tree, walnut tree, beech tree, pine tree, etc.

Without wishing to be bound by any theory, it has been described that biofilm formation by plant growth promoting microorganisms on the roots of plants provides them with greater protection against abiotic stress situations such as, for example, adverse weather conditions, changes in temperature, and desiccation or water stress conditions (e.g. high or low temperatures, drought, etc.) (Seneviratne et al., 2011, Soil Biol. Biochem. 43:1059-1062). Additionally, the absorption of amino acids by the plant provides the plant with greater resistance to abiotic stresses (Triveni et al., 2012, Annals Microbiol. 63:1147-1156; Lucas et al., 2014, Plant Physiol. Biochem. 82:44-53).

### 3.3. Methods for solubilising insoluble compounds

### 3.3.1. Method for solubilising insoluble phosphates in a solid substrate

In another aspect, the present invention relates to a method for solubilising an insoluble phosphate in a solid substrate, hereinafter "the method for solubilisinq an insoluble phosphate in a solid substrate of the invention", comprising the step of applying at least one variant strain according to the invention to the solid substrate.

In an embodiment, at least two, or at least three, or at least 4, or at least 5 different strains of the invention are applied to the solid substrate.

Alternatively, the method for solubilising an insoluble phosphate in a soil of the invention comprises the step of applying the supernatant of the invention to the soil.

It will be readily appreciated that the variant strain of the invention may be applied in different ways, i.e. directly to the solid substrate by injection, or as part of a fertiliser, adhered to a substrate or solid support, or as part of a soil.

Alternatively, the method for solubilising an insoluble phosphate in a solid substrate of the invention may comprise the step of applying the fertiliser of the invention to the solid substrate.

Alternatively, the method for solubilising an insoluble phosphate in a solid substrate of the invention may comprise the step of applying the substrate of the invention to the solid substrate.

Alternatively, the method for solubilising an insoluble phosphate in a solid substrate of the invention may comprise the step of applying the active solid support of the invention to the solid substrate.

Alternatively, the method for solubilising an insoluble phosphate in a solid substrate of the invention may comprise the step of applying the soil of the invention to the solid substrate.

The terms "variant strain of the invention", "supernatant of the invention", "fertiliser of the invention", "substrate of the invention", "active solid support of the invention", and "soil of the invention", "have been described previously in detail and their definitions and embodiments are included here by reference.

The solid substrate may contain at least one source of an insoluble phosphate. Virtually any solid substrate containing an insoluble phosphate can be treated according to this process for completely or partially reducing the insoluble phosphate content; nevertheless in a particular embodiment, said solid substrate containing an insoluble phosphate to be treated is rock phosphate, such as the rock phosphate used to produce phosphate fertilisers with a variable insoluble phosphate content.

This process can be used to solubilise all or part of the insoluble phosphate present in the solid substrate containing an insoluble phosphate, for example rock phosphate used for producing phosphate fertilisers, for the purpose of reducing the insoluble phosphate content present in said phosphate fertilisers obtained from said rock phosphate.

The solid substrate containing an insoluble phosphate to be treated may be mixed with a culture of the variant strain of the invention, or with a substrate of the invention, or with an active solid support of the invention, or with soil of the invention, at a bacterial population density which may vary within a wide range. In a particular embodiment, at least 10 cfu per ml of water, at least 10² cfu per ml of water, at least 10³ cfu per ml of water, at least 10⁴ cfu per ml of water, at least 10⁵ cfu per ml of water, at least 10⁶ cfu per ml of water, at least 10⁷ cfu per ml of water, at least 10⁸ cfu per ml of water, at least 10⁹ cfu per ml of water, or more are inoculated.

Following inoculation, the strain of the invention is left to act for at least 1 hour, at least 2 hours, at least 3 hours, at least 4 hours, at least 5 hours, at least 10 hours, at least 15 hours, at least 20 hours, at least 24 hours, or longer. The incubation is preferably done under aerobic conditions. If desired, the strain of the invention is removed by conventional methods, such as by means of decantation, precipitation with electrolytes, centrifugation, etc. If desired, the removed microorganisms of the strain of the invention can be reused in a new method of solubilisation of an insoluble phosphate and/or insoluble iron, in a solid substrate or in a soil containing an insoluble phosphate and/or insoluble iron.

This process can be carried out in a reactor, pond or the like in which the solid substrate containing an insoluble phosphate to be treated is introduced, duly equipped with means for feeding and draining water, solid substrate, inoculation of microorganisms of the invention and recovery of the same. If necessary, the mixture (solid substrate, water and variant strain of the invention optionally forming part of a fertiliser, substrate, active solid support or soil of the invention) is supplemented with a carbon source and/or with a nitrogen source and/or essential nutrients, for the purpose of facilitating survival of the variant strain of the invention. By way of illustration, suitable amounts of micronutrient solution along with suitable amounts of magnesium, cobalt and molybdenum, typically in the micromolar order, can be added to optimize the process; nevertheless, in any case the choice and amount of nutrients and micronutrients to be added will depend on the composition of the solid substrate containing an insoluble phosphate (e.g., rock phosphate) to be treated and on the microbiological demand.

### 3.3.2. Method for solubilising insoluble phosphates in a soil

In another aspect, the present invention relates to a method for solubilising insoluble phosphates in a soil, hereinafter "the method for solubilisinq insoluble phosphates in a soil of the invention", comprising the step of applying at least one variant strain according the invention on the soil.

In an embodiment, an effective amount of at least two, at least three, at least 4 or at least 5 different strains of the invention are applied to the soil.

Alternatively, the method for solubilising an insoluble phosphate in a soil of the invention comprises the step of applying the supernatant of the invention to the soil.

The person skilled in the art will appreciate that the solubilisation treatment of the soil containing insoluble phosphate may be carried out in a variety of different ways. For example, the step of applying at least one variant strain according the invention on the soil to be treated may be carried out by injecting a culture of at least one of the strains of the invention into the soil to be treated; or by adding to said soil to be treated a supplemented fertiliser of the invention, or by admixing into said soil to be treated an active solid support of the invention; or by sowing a seed of the invention or a root ball of the invention in said soil to be treated; or by admixing into said soil to be treated a soil according to the invention.

Thus, the method for solubilising an insoluble phosphate in a soil of the invention may comprise a step of applying the fertiliser of the invention to the soil.

Alternatively, the method for solubilising an insoluble phosphate in a soil of the invention may comprise a step of applying the substrate of the invention to the soil. Alternatively, the method for solubilising an insoluble phosphate in a soil of the invention may comprise a step of applying the soil of the invention to the soil. The soil of the invention may have the same composition as the soil to be treated, except for the content in the strain of the invention, or a different composition to the soil to be treated.

Alternatively, the method for solubilising an insoluble phosphate in a soil of the invention may comprise a step of sowing a seed of the invention or a root ball of the invention in said soil to be treated.

Alternatively, the method for solubilising an insoluble phosphate in a soil of the invention may comprise a step of sowing a root ball of the invention in said soil to be treated.

The method for solubilising an insoluble phosphate in a soil may be carried out under aerobic conditions.

The particular amount of insoluble phosphate present in the soil may make it necessary to re-apply the strain of the invention, regardless of the form of presentation. Thus, the step of applying at least one variant strain according the invention on the soil may be carried out once, twice, three times, four times, five times, 6 times, 7 times, 8 times, 9 times, 10 times, or more, until partial or complete solubilisation of the insoluble phosphate is achieved.

Virtually any soil containing an insoluble phosphate, such as dibasic calcium phosphate, tribasic calcium phosphate, or mixtures of different insoluble phosphates, can be treated according to this method in order to completely or partially reduce the insoluble phosphate content present in the soil. In an embodiment, said soil is an agricultural topsoil, preferably a topsoil treated, for example, with fertilisers.

If necessary, the soil to be treated is supplemented with a carbon source and/or nitrogen source and/or essential nutrients to facilitate survival of the variant strain of the invention.

The injection of the at least one variant strain of the invention is done for the purpose of achieving a high cell density in the soil to be treated, for example, of at least 10³ cfu per gram of soil to be treated, or of at least than 10⁴ cfu per gram of soil to be treated, or of at least than 10⁵ cfu per gram of soil to be treated, or of at least than 10⁶ cfu per gram of soil to be treated, or of at least than 10⁷ cfu per gram of soil to be treated.

In a particular embodiment, said method for the solubilisation of insoluble phosphate present in a soil containing an insoluble phosphate is carried out by means of adding to said soil to be treated a substrate of the invention, comprising a variant strain of the invention. Features of said substrate of the invention have previously been described. The amount of substrate of the invention which is added to the soil to be treated (soil containing an insoluble phosphate) may vary within a broad range. Nevertheless, in a particular embodiment the amount of said substrate of the invention which is added to the soil to be treated is equal to or greater than 0.01 kg of substrate of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble phosphate.

In a particular embodiment, said method for the solubilisation of insoluble phosphate present in a soil containing an insoluble phosphate is carried out by means of adding to said soil to be treated an active solid support of the invention, comprising a variant strain of the invention. Features of said active solid support of the invention have previously been described. The amount of active solid support of the invention which is added to the soil to be treated (soil containing an insoluble phosphate) may vary within a broad range. Nevertheless, in a particular embodiment the amount of said active solid support of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble phosphate.

In a particular embodiment, said method for the solubilisation of insoluble phosphate present in a soil containing an insoluble phosphate is carried out by means of adding to said soil to be treated a supplemented soil of the invention, comprising a variant strain of the invention. Features of said soil of the invention have previously been described. The amount of soil of the invention which is added to the soil to be treated (soil containing an insoluble phosphate) may vary within a broad range. Nevertheless, in a particular embodiment the amount of said soil of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble phosphate.

In another particular embodiment, said method for the solubilisation of insoluble phosphate present in a soil containing an insoluble phosphate is carried out by means of adding to said soil to be treated a fertiliser of the invention comprising a variant strain of the invention. The features of said fertiliser of the invention have been previously described. The amount of fertiliser of the invention which is added to the soil to be treated (soil containing an insoluble phosphate) may vary within a wide range, depending on, among other factors, the amount of insoluble phosphate present in the soil to be treated and on the amount of variant strain of the invention present in the fertiliser of the invention.

In another particular embodiment, the method for the solubilisation of insoluble phosphate present in a soil containing insoluble phosphate is carried out by means of sowing said soil to be treated with a seed of the invention comprising a variant strain of the invention. The features of said seed of the invention have been previously described. The amount of supplemented seeds of the invention which are sowed in the soil to be treated (soil containing insoluble phosphate) may vary within a wide range, depending on the density required by the farmer.

In another particular embodiment, the method for the solubilisation of insoluble phosphate present in a soil containing insoluble phosphate is carried out by means of sowing said soil to be treated with a root ball of the invention comprising a variant strain of the invention. The features of said root ball of the invention have been previously described. The amount of supplemented root balls of the invention which are sown in the soil to be treated (soil containing insoluble phosphate) may vary within a wide range, depending on the density required by the farmer.

The methods of solubilisation of insoluble phosphates described above provide a number of advantages, including the following:
- high specificity in the solubilisation of insoluble phosphates;
- they work in a broad range of phosphate concentrations, typically between 0.01% and 95% (w/w) of phosphate; and
- they are highly versatile since they can be used *in situ* for solubilising said compounds in soils or in reactors for solubilising phosphate, which can be used as fertiliser *per se* or in mixtures with other compounds.

### 3.3.3. Method for solubilising iron in a solid substrate

In another aspect, the present invention relates to a method for solubilising insoluble iron in a solid substrate, hereinafter "the method for solubilising insoluble iron in a solid substrate of the invention", comprising the step of applying at least one variant strain according the invention on the solid substrate.

In an embodiment, an effective amount of at least two, at least three, at least 4 or at least 5 different strains of the invention are applied to the solid substrate.

Alternatively, the method for solubilising insoluble iron in a solid substrate of the invention comprises the step of applying the supernatant of the invention to the solid substrate.

It will be readily appreciated that the variant strain of the invention may be applied in different ways, i.e. directly to the solid substrate, or as part of a fertiliser, adhered to a solid support, or as part of a soil.

Alternatively, the method for solubilising insoluble iron in a solid substrate of the invention may comprise the step of applying the fertiliser of the invention to the solid substrate.

Alternatively, the method for solubilising insoluble iron in a solid substrate of the invention may comprise the step of applying the substrate of the invention to the solid substrate.

Alternatively, the method for solubilising insoluble iron in a solid substrate of the invention may comprise the step of applying the solid support of the invention to the solid substrate.

Alternatively, the method for solubilising insoluble iron in a solid substrate of the invention may comprise the step of applying the soil of the invention to the solid substrate.

The terms "variant strain of the invention", "supernatant of the invention", "fertiliser of the invention", "substrate of the invention", "active solid support of the invention", and "soil of the invention", have been described previously in detail and their definitions and embodiments are included here by reference.

The solid substrate may contain at least one source of insoluble iron. Virtually any solid substrate containing an insoluble phosphate (for example, insoluble iron of the iron oxide type or any other form of iron) can be treated according to this method in order to completely or partially reduce the insoluble phosphate content. Non-limiting examples of solid substrates containing an insoluble phosphate include rock phosphate, such as rock phosphate used for producing phosphate fertilisers with a variable insoluble phosphate content, and a soil, such as an agricultural topsoil, a topsoil treated with fertilisers, etc.

The method may optionally comprise a step of removing the microorganisms of the variant strain of the invention.

For carrying out this method, the solid substrate containing insoluble iron to be treated is mixed with water and the resulting mixture is put in contact with a culture of one of the variant strains of the invention, or with a fertiliser of the invention, or with a substrate of the invention, or with an active solid support of the invention, at a bacterial population density that may vary within a broad range. In a particular embodiment, at least 10³ cfu of the variant strain of the invention per ml of water are inoculated. After the inoculation, the variant strains of the invention are left to act, and at the end of 1 to 24 hours, if desired, said microorganisms are removed by conventional methods, for example, by means of decantation, precipitation with electrolytes, centrifugation, etc. If desired, the removed variant strains of the invention can be reused in a new method of microbiological solubilization, under aerobic conditions, of insoluble phosphate and/or insoluble iron in a solid substrate or in a soil containing insoluble phosphate and/or insoluble iron.

This method can be carried out in a reactor, pool, or the like, in which the solid substrate containing insoluble iron to be treated is introduced, and which is duly equipped with means for the feeding in and discharging of water, solid substrate, the inoculation of variant strains of the invention, and the recovery thereof. If necessary, the mixture (solid substrate, water, and variant strains of the invention that are optionally part of a substrate or an active solid support of the invention) is supplemented with a carbon source and/or with a nitrogen source and/or essential nutrients for the purpose of facilitating survival of the variant strains of the invention. By way of illustration, suitable amounts of a micronutrient solution together with suitable amounts of magnesium, cobalt and molybdenum, typically in the micromolar order, can be added to optimize the method. Nevertheless, the selection and amount of nutrients and micronutrients to be added would depend, in any case, on the composition of the solid substrate containing insoluble iron to be treated and on the microbiological demand.

This method can be used for solubilising all or part of the insoluble iron present in the solid substrate containing insoluble iron for the purpose of reducing the insoluble iron content present in said solid substrate.

### 3.3.4. Method for solubilising iron in a soil

In another aspect, the invention relates to a process for the solubilisation of insoluble iron present in a soil containing insoluble iron, hereinafter "the method for solubilising insoluble iron in a soil of the invention", comprising contacting said soil containing insoluble iron with a variant strain of the invention.

In an embodiment, an effective amount of at least two, at least three, at least 4 or at least 5 different strains of the invention are applied to the soil containing insoluble iron.

Alternatively, the method for solubilising insoluble iron in a soil of the invention comprises the step of applying the supernatant of the invention to the soil containing insoluble iron.

The person skilled in the art will appreciate that the solubilisation treatment of the soil containing insoluble iron may be carried out in a variety of different ways. For example, the step of applying at least one variant strain according the invention on the soil to be treated may be carried out by injecting a culture of at least one of the strains of the invention into the soil to be treated; or by adding to said soil to be treated a supplemented fertiliser of the invention, or by admixing into said soil to be treated an active solid support of the invention; or by sowing a seed of the invention or a root ball of the invention in said soil to be treated; or by admixing into said soil to be treated a soil according to the invention.

Alternatively, the method for solubilising insoluble iron in a soil of the invention may comprise the step of applying the fertiliser of the invention to the solid substrate.

Alternatively, the method for solubilising insoluble iron in a soil of the invention may comprise the step of applying the substrate of the invention to the solid substrate.

Alternatively, the method for solubilising insoluble iron in a soil of the invention may comprise the step of applying the solid support of the invention to the solid substrate.

Alternatively, the method for solubilising insoluble iron in a soil of the invention may comprise the step of applying the soil of the invention to the solid substrate.

The terms "variant strain of the invention", "supernatant of the invention", "fertiliser of the invention", "substrate of the invention", "active solid support of the invention", "soil of the invention", "seed of the invention", and "root ball of the invention", have been described previously in detail and their definitions and embodiments are included here by reference.

The particular amount of insoluble iron present in the soil may make it necessary to re-apply the strain of the invention, regardless of the form of presentation. Thus, the step of applying at least one variant strain according the invention on the soil may be carried out once, twice, three times, four times, five times, 6 times, 7 times, 8 times, 9 times, 10 times, or more, until partial or complete solubilisation of the insoluble iron is achieved.

Virtually any soil containing insoluble iron (e.g., insoluble iron of the iron oxide type or any another form of iron) can be treated according to this process for removing all or part of the insoluble iron for completely or partially reducing the insoluble iron content present in the soil to be treated; nevertheless in a particular embodiment, said soil containing insoluble iron to be treated is a soil, such as an agricultural topsoil, a topsoil treated with fertilisers, etc. If necessary, the soil to be treated is supplemented with a carbon source and/or with a nitrogen source and/or with essential nutrients to facilitate survival of the microorganisms of the variant strain of the invention.

The injection of the at least one variant strain of the invention is done for the purpose of achieving a high cell density in the soil to be treated, for example, of at least 10³ cfu per gram of soil to be treated, or of at least than 10⁴ cfu per gram of soil to be treated, or of at least than 10⁵ cfu per gram of soil to be treated, or of at least than 10⁶ cfu per gram of soil to be treated, or of at least than 10⁷ cfu per gram of soil to be treated.

In a particular embodiment, said method for the solubilisation of insoluble iron present in a soil containing an insoluble iron is carried out by means of adding to said soil to be treated a substrate of the invention, comprising a variant strain of the invention. Features of said substrate of the invention have previously been described. The amount of substrate of the invention which is added to the soil to be treated (soil containing an insoluble iron) may vary within a broad range. Nevertheless, in a particular embodiment the amount of said substrate of the invention which is added to the soil to be treated is equal to or greater than 0.01 kg of substrate of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble iron.

In a particular embodiment, said method for the solubilisation of insoluble iron present in a soil containing an insoluble iron is carried out by means of adding to said soil to be treated an active solid support of the invention, comprising a variant strain of the invention. Features of said active solid support of the invention have previously been described. The amount of active solid support of the invention which is added to the soil to be treated (soil containing an insoluble iron) may vary within a broad range. Nevertheless, in a particular embodiment the amount of said active solid support of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble iron.

In a particular embodiment, said method for the solubilisation of insoluble iron present in a soil containing an insoluble iron is carried out by means of adding to said soil to be treated a supplemented soil of the invention, comprising a variant strain of the invention. Features of said soil of the invention have previously been described. The amount of soil of the invention which is added to the soil to be treated (soil containing an insoluble iron) may vary within a broad range. Nevertheless, in a particular embodiment the amount of said soil of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble iron.

In another particular embodiment, said method for the solubilisation of insoluble iron present in a soil containing an insoluble iron is carried out by means of adding to said soil to be treated a fertiliser of the invention comprising a variant strain of the invention. The features of said fertiliser of the invention have been previously described. The amount of fertiliser of the invention which is added to the soil to be treated (soil containing an insoluble iron) may vary within a wide range, depending on, among other factors, the amount of insoluble iron present in the soil to be treated and on the amount of variant strain of the invention present in the fertiliser of the invention.

In another particular embodiment, the method for the solubilisation of insoluble iron present in a soil containing insoluble iron is carried out by means of sowing said soil to be treated with a seed of the invention comprising a variant strain of the invention. The features of said seed of the invention have been previously described. The amount of supplemented seeds of the invention which are sown in the soil to be treated (soil containing insoluble iron) may vary within a wide range, depending on the density required by the farmer.

In another particular embodiment, the method for the solubilisation of insoluble iron present in a soil containing insoluble iron is carried out by means of sowing said soil to be treated with a root ball of the invention comprising a variant strain of the invention. The features of said root ball of the invention have been previously described. The amount of supplemented root balls of the invention which are sown in the soil to be treated (soil containing insoluble iron) may vary within a wide range, depending on the density required by the farmer.

The solubilisation of iron, for example, by means of chelation by siderophores, prevents the iron from being available for pathogenic microorganisms, particularly plant pathogens, thus exerting biocontrol on agriculturally unwanted microbial populations. This advantage can be obtained by means of the use of the variant strain of the invention.

The present invention also contemplates methods for solubilising insoluble phosphates and insoluble iron in a solid substrate or a soil comprising insoluble phosphates and iron, comprising the step of applying at least one variant strain according to the invention to the solid substrate or soil. The skilled person will appreciate that this method is a combination of the methods for solubilising insoluble phosphates and the methods for solubilising insoluble iron that have been described previously.

### 4. Method for obtaining a variant strain according to the invention

The variant strains of the invention may be generated from existing strains by mutagenesis. It is particularly advantageous that the starting strains exhibit one or more of the characteristics of the strain of the invention.

Thus, in another aspect, the present invention also relates to a method for obtaining a strain with capacity to produce at least one plant growth-stimulating compound and/or to solubilise insoluble phosphate and/or iron, hereinafter "the first method of mutagenesis of the invention", comprising the steps of:
(i) mutagenising *P. putida* BIRD-1 strain with accession number CECT 7415 in the presence of an analogue of the growth-stimulating compound and
(ii) selecting a strain which are capable of growing in the presence of an analogue of the growth-stimulating compound.

It will be appreciated that the strains of the invention may be improved by mutagenesis to obtain additional desirable properties, such as the capacity to produce additional plant growth-stimulating compounds. In addition, the first method of mutagenesis of the invention may further comprise determining whether the mutated strain maintains the capacity of the parent strain to solubilise insoluble phosphate and/or to solubilize iron. This determination can be carried out using the methods disclosed in Example 1.

Thus, in another aspect, the present invention also relates to a method for obtaining a strain with capacity to produce at least one plant growth-stimulating compound, hereinafter "the second method of mutagenesis of the invention", comprising the steps of:
(i) mutagenising a variant strain according to the invention in the presence of an analogue of the growth-stimulating compound and
(ii) selecting a strain which is capable of growing in the presence of an analogue of the growth-stimulating compound.

In a preferred embodiment, the method for obtaining a strain with capacity to produce at least one plant growth-stimulating compound further comprises an additional step which comprises searching for a clone that overproduce amino acids. The selection step can be carried out either using cross-feeding assays or by chemical analysis of amino acids in culture supernatants. In addition, the second method of mutagenesis of the invention may further comprise determining whether the mutated strain maintains the capacity of the parent strain to solubilise insoluble phosphate and/or to solubilize iron. This determination can be carried out using the methods disclosed in Example 1. The terms "strain", "variant strain of the invention", "capacity to produce at least one plant growth-stimulating compound", "capacity to solubilise insoluble phosphate", "capacity to solubilise iron", and *"P. putida* BIRD-1 strain with accession number CECT 7415", have been described previously in detail and their definitions and embodiments are included here by reference.

The first and second methods of mutagenesis of the invention only differ in the starting strain. Accordingly, the embodiments describes hereinafter apply equally to the first and second methods of mutagenesis of the invention.

In a first step, the first and second methods of mutagenesis of the invention comprise a step of mutagenizing CECT 7415 *P. putida* BIRD-1 or a variant strain according to the invention in the presence of an analogue of the growth-stimulating compound.

The term "mutagenesis", as used herein, refers to a process by which the genetic information of an organism is changed, resulting in a mutation. Mutagenesis may be random mutagenesis or site-directed mutagenesis. Random mutagenesis results in completely random mutations whereas site-directed mutagenesis makes specific and intentional changes to the DNA sequence of a gene.

In a particular embodiment, the mutagenesis is random mutagenesis. This may be achieved by exposing microorganisms of the starting strains to mutagens such as electromagnetic radiation and/or ionising radiation, such as UV radiation, X-rays or gamma-rays, or to mutagenic chemicals, such as alkylating agents. The resulting mutants are then selected for the desired characteristics.

In an embodiment, the mutagenesis is achieved by exposing microorganisms of the starting strains to an alkylating agent. Non-limiting examples of alkylating agent that are useful for the purposes of the invention include ethyl methanesulfonate (EMS) and N-ethyl-N-nitrosourea (ENU). The mutagenesis using an alkylating agent may be carried out by any conventional method. For example, the method may use plates containing the microorganism of the starting strain and a concentration equivalent to the minimum inhibitory concentration (MIC) or higher for said microorganism of the analogue of the growth-stimulating compound whose production is sought. The microorganisms are put into contact with the alkylating agent, e.g. EMS or ENU, by means of depositing a filter containing a suitable amount of EMS or EMU on the plates. Following at, for example, 30°C for 48 h, an inhibition halo is formed on the filter that faded as the concentration of the mutagenic agent decreased due to the mutagenic effect of EMS. Colonies that appear within the inhibition halo are potential mutants that produce the growth-stimulating compound whose production is sought. Individual colonies are then transferred to a suitable medium, such as M9 minimal medium, for further screening.

In a particular embodiment, the growth-stimulating compound is an L-amino acid. Analogues of L-amino acids are well known to the person skilled in the art. Non-limiting examples of analogues of growth-stimulating compounds include 5-fluoro-DL-tryptophan, a tryptophan analogue; *p*-fluoro-DL-phenylalanine, a phenylalanine analogue; L-norvaline, a valine/isoleucine analogue; and L-methionine-D,L-sulfoximine (MSX), a glutamate/glutamine analogue.

In the case of 5-fluoro-DL-tryptophan, a tryptophan analogue, a concentration equal to or greater than 1.5 mg/L, was added. All the possible mutants secreting tryptophan were transferred to plates containing M9 minimal medium, as previously described.

In another particular embodiment, the first step is performed in a culture medium comprising an analogue of the growth-stimulating compound and one or more of the following: a carbon source, a nitrogen source, a phosphorus source, and micronutrients.

The mutants obtained in the first step are then screened for the capacity of producing the particular growth-stimulating compound.

In a second step, the methods comprise a step of selecting strains which are capable of growing in the presence of an analogue of the growth-stimulating compound.

This step may be carried out by any conventional means, such as by incubating the strains obtained in the first step in the presence of increasing amounts of the analogue of the growth-stimulating compound and monitoring growth.

The strains which have the capacity to grow in the presence of the analogue of the growth-stimulating compound do so because they produce said compound, i.e. they are auxotrophic for that compound. The selected strains may be further screened for additional properties, such as the capacity to produce at least one additional plant growth stimulating compound, the capacity to solubilise insoluble phosphate, and the capacity to solubilise insoluble iron. This may be achieved by means of the methods described in the context of the variant strain of the invention.

The present invention also contemplates the strain obtainable by the first and second methods of mutagenesis of the invention.

### 5. Uses

In another aspect, the present invention relates to the use of the variant strain according to the invention, or the supernatant according to the invention, or the fertiliser, seed, root ball, substrate, active solid support, or soil according to the invention, for stimulating plant growth.

In another aspect, the present invention relates to the use of the variant strain according to the invention, or the supernatant according to the invention, or the fertiliser, seed, root ball, substrate, active solid support, or soil according to the invention, for restoring the normal state of a plant after suffering environmental stress.

In another aspect, the present invention relates to the use of the variant strain according to the invention, or the supernatant according to the invention, or the fertiliser, seed, root ball, substrate, active solid support, or soil according to the invention, for obtaining a variant strain with capacity to produce at least one plant growth-stimulating compound.

In another aspect, the present invention relates to the use of the variant strain according to the invention, or the supernatant according to the invention, or the fertiliser, seed, root ball, substrate, active solid support, or soil according to the invention, to solubilise insoluble phosphate.

In another aspect, the present invention relates to the use of the variant strain according to the invention, or the supernatant according to the invention, or the fertiliser, seed, root ball, substrate, active solid support, or soil according to the invention, to solubilise iron.

In another aspect, the present invention relates to the use of the variant strain according to the invention, or the supernatant according to the invention, or the fertiliser, seed, root ball, substrate, active solid support, or soil according to the invention, to solubilise insoluble phosphate and iron.

### BIOLOGICAL MATERIAL DEPOSITS

The *Pseudomonas putida* BIRD-1-12 strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 14 November 2018, and the number assigned to said deposit was CECT 9761.

The *Pseudomonas putida* BIRD-1-12F strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 14 November 2018, and the number assigned to said deposit was CECT 9762.

The *Pseudomonas putida* BIRD-1-12S strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 14 November 2018, and the number assigned to said deposit was CECT 9763.

The *Pseudomonas putida* BIRD-1-21 strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 14 November 2018, and the number assigned to said deposit was CECT 9764.

The Pseudomonas putida BIRD-1 strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 3 June 2018, and the number assigned to said deposit was CECT 7415.

These aspects of the invention exploit the use of the variant strain according to the invention, or the supernatant according to the invention, or the fertiliser, seed, root ball, substrate, active solid support, or soil according to the invention in the methods of the invention described previously.

### EXAMPLES

### Example 1: Determination of the capacity to produce plant hormones and compounds with the capability to make proteic nitrogen bioavailable for CECT 7415 P. putida BIRD-1 strain

The capacity to produce plant hormones and compounds with the capability to make protein nitrogen bioavailable for CECT 7415 *P. putida* BIRD-1 strain was determined using a number of different solid media.

### 1. Production of plant hormones or plant hormone promoting compounds, such as indoleacetic acid (IAA):

An amended LB medium was used, the specific composition per liter of which is: 10 g bacto-tryptone, 5 g yeast extract, 10 g NaCl, 1.025 g L-tryptophan, 600 mg sodium dodecylsulfate (SDS), 10 mL glycerol, and 15 g agar. To conduct this assay, a no. 1 Whatman paper disc was placed above the medium with the inoculated CECT 7415 *P. putida* BIRD-1 and it was left to incubate. Once incubation ended, the Whatman paper was reacted with Salkowski reagent (Naik *et al.,* 2008, as above), the specific composition per litre of which is: 2% (v/v) of a solution of 0.5 M FeCl₃ in a 35% (v/v) perchloric acid solution. The Whatman paper turned a reddish color after 1 h of incubation and thus it was considered positive for IAA production.

### 2. Production of siderophores:

A specific medium referred to as CAS Agar was used (Alexander & Zuberer, 1991, Biology Fertility Soils 12:39) to confirm the production of siderophores, i.e. iron chelating substances which allow its assimilation by plants. The specific composition per liter of CAS Agar is: 50 ml of CAS solution (Chrome Azurol S) (1.21 mg/ml), 10 ml of FeCl₃ x 6H₂O (1 mM) dissolved in 10 mM HCI 10, 40 ml of hexadecyltrimethylammonium (HDTMA) (1.82 mg/ml), 30.24 g of piperazine-1.4-bis (PIPES), 0.3 g of KH₂PO₄, 0.5 g of NaCl, 1 g of NH₄Cl, 2 g of glucose, 2 g of mannitol, 493 mg of MgSO₄ x 7H₂O, 12.5 mg of CaCl₂ x 2 H₂O, 1.17 mg of MnSO₄ x 2 H₂O, 1.4 mg of H₃BO₃, 0.04 mg of CuSO₄ x 5H₂O, 1.2 mg of ZnSO₄ x 7H₂O, 1.08 mg of Na₂MoO₄ x 2 H₂O and 3 mg of casamino acids. It was considered positive for the production of siderophores since a yellow halo was observed in the medium around the colony of CECT 7415 *P. putida* BIRD-1.

### 3. Solubilisation of insoluble phosphates:

A specific medium referred to as Agar Pikovskaya (Naik *et al.,* 2008, as above), was used to confirm that CECT 7415 *P. putida* BIRD-1 solubilises insoluble phosphates. The specific composition per liter of Agar Pikovskaya is: 0.5 g of yeast extract, 10 g of glucose, 5 g of Ca₃(PO₄)₂, 0.5 g of (NH₄)₂SO₄, 0.2 g of KCI, 0.1 g of MgSO₄ x 7H₂O, 0.1 mg of MnSO₄ x 2 H₂O, 0.1 mg of FeSO₄ x 7 H₂O, and 15 g of agar. It was considered positive for the production of phosphatases since a clear halo was produced in the medium around the colony.

### 4. Solubilisation of phytate:

Phytate is the most abundant organic form of phosphorus in plant material. In order to confirm that CECT 7415 *P. putida* BIRD-1 solubilizes phytate, growth of the strain on phytate agar medium (Husseinkhani et al., 2009, African J Biotechnol 8:4229-4232) was estimated its composition being: 10 g glucose, 4 g sodium phytate, 2 g Ca Cl₂, 5g NH₄NO₃, 0.5 g KCI, 0.5g Mg SO₄ x 7 H₂O, 0.01 g MnSO₄ x H₂O and 15 g agar. The positive clones produced a clear halo around the colony.

High efficiency was found in the above procedures since the CECT 7415 *P. putida* strain BIRD-1 yielded a positive reaction in less than 24-48 h.

### Example 2: Obtaining a mutant microorganism producing tryptophan derived from CECT 7415 P. putida BIRD-1

The CECT 7415 *P. putida* BIRD-1 strain (Example 1) was subjected to a process of random mutagenesis in which said microorganism was put in contact with ethylmethanesulfonate (EMS), a mutagenic agent, in an M9 medium (Abril et al., 1989, J Bacteriology 171:6782), the specific composition per litre of which was: 6.4 g of Na₂HPO₄ x 7H₂O, 1.5 g of KH₂PO₄, 0.25 g of NaCl, 0.5 g of NH₄Cl, 1 mL of 1 M MgSO₄, 1 ml of 6% (w/w) ferric citrate, 25 mL of 20% (w/v) glucose, and 15 g of agar. A concentration equivalent to the minimum inhibitory concentration (MIC) for said CECT 7415 *P. putida* BIRD-1 of 5-fluoro-DL-tryptophan, a tryptophan analogue, at a concentration equal to or greater than 1.5 mg/L, was added. Briefly, a filter containing 10 mg of EMS was deposited on the plates containing said microorganism, and it was incubated at 30°C for 48 h. Due to the mutagenic effect of EMS, an inhibition halo was formed on the filter that faded as the concentration of the mutagenic agent decreased. Colonies appeared within the inhibition halo and these colonies were potential mutants that produced excess tryptophan, which highlighted the inhibitory effect of the amino acid analogue. All the possible mutants overproducing tryptophan were transferred to plates containing M9 minimal medium.

### Example 3: Assays for determining resistance to 5-fluoro-DL-tryptophan

The colonies of mutants overproducing tryptophan resulting from Example 2 were seeded in M9 minimal medium supplemented with three different concentrations of 5-fluoro-DL-tryptophan, i.e. 1.5 mg/mL, 3 mg/mL, and 5 mg/mL. The thirty clones that grew in the presence of 3 mg/ml and of these four clones grew in the presence of 5 mg/mL of the amino acid analogue (5-fluoro-DL-tryptophan), these thirty clones were selected as mutants resistant to 5-fluoro-DL-tryptophan and susceptible to overcoming the toxic effect via production of the amino acid.

### Example 4: Confirming the capacity of production of tryptophan by the mutants resistant to 5-fluorotryptophan.

*P. putida* Δ*trpA* PP0082 is a mutant that was described as having a mini-Tn*5* insertion at the *trpA* gene in the tryptophan biosynthetic pathway (Molina-Henares et al., 2010, Environ. Microbiol. 12:1468-1485). This mutant strain fails to grow on minimal medium unless tryptophan is added to the medium. To check if the thirty mutants selected in example 3 were able to produce tryptophan, a cross-feeding assay was performed on 20 x 20 cm plates with M9 minimal medium (Abril et al, 1989, J. Bacteriol 171:6782-6794). Drops of each of the 5-fluorotryptophan resistant mutants were deposited with a 1 cm separation in the Y axis of the plate and with a 5 cm space in the X axis. Once the drops were dried, the *trpA* mutant was picked with a toothpick at 5, 10,15, 20, 25 mm from the drop both at the right and the left of the drop. With this array we expected to determine whether 5-fluorotryptophan resistance was due to the overproduction of the amino acid tryptophan. If the *trpA* mutant grew it was considered indicative of the production of L-tryptophan and the higher the concentration of the amino acid produced the furthest the growth of the *trpA* mutant with respect to the drop. These clones were named BIRD-1-12, BIRD-1-21, BIRD1-27 and BIRD-1-30. Two of the clones, the one named *P. putida* BIRD-1-21 with accession number CECT 9764 and *P. putida* BIRD-1-12 with accession number CECT 9761 - were selected for further assays because they allowed cross-feeding to *trpA* cells located 20mm away from the drop, while the others three allowed growth up to 15. CECT 9761 *P. putida* BIRD-1-12 was selected for another round of mutagenesis in order to obtain mutants overproducing other amino acids such as phenylalanine, for example.

### Example 5: Obtaining a mutant microorganism overproducing phenylalanine derived from CECT 9761 P. putida BIRD-1-12

The CECT 9761 *P. putida* BIRD-1-12 strain obtained in Example 4, selected as a good tryptophan producer, was subjected to a method of random mutagenesis similar to the one described in Example 2, but using *p*-fluoro-DL-phenylalanine as the amino acid analogue (phenylalanine). Briefly, a filter containing 10 mg of EMS was deposited on the plates containing CECT 9761 *P. putida* BIRD-1-12 in M9 medium supplemented with 1000 µg/ml p-fluoro-DL-phenylalanine, and it was incubated at 30°C for 48 h. Due to the mutagenic effect of EMS, an inhibition halo was formed on the filter that faded as the concentration of the mutagenic agent decreased. Colonies appeared within the inhibition halo and these colonies were potential mutants that produced excess phenylalanine, which highlighted the inhibitory effect of the amino acid analogue. All the possible mutants overproducing phenylalanine were transferred to plates containing M9 minimal medium.

### Example 6: Assays for determining resistance to p-fluoro-DL-phenylalanine

The colonies resulting from Example 5 were seeded in plates containing M9 minimal medium supplemented with 1 mg/mL, 2 mg/mL, 3 mg/mL and 5 mg/mL of *p*-fluoro-DL-phenylalanine. Those clones that grew in the presence of the highest concentration of *p*-fluoro-DL-phenylalanine were selected as mutants resistant to *p*-fluoro-DL-phenylalanine and susceptible to overcoming the toxic effect via production of the amino acid. Twelve mutants that were able to grow in the presence of > 3mg/mL of the amino acid analogue were selected as potential phenylalanine producing strains.

### Example 7. Cross-feeding of phenylalanine auxotroph of P. putida by mutant variants of BIRD-1-12 that are resistance to p-fluorophenylanine.

*P. putida* Δ*pheA* (PP1769) is a mutant that was described as having a mini-Tn*5* insertion at the *pheA* gene in the phenylalanine biosynthetic pathway. This mutant strain fails to grow on minimal medium unless a concentration over 10 micromolar of phenylalanine is added to the medium. To check if the twelve mutants selected in example 6 were able to produce phenylalanine, a cross-feeding assay was performed on 20 x 20 cm plates of M9 minimal medium (Abril *et al,* 1989., cited above), as described in Example 4 except that instead of using the *trpA* mutant, the *pheA* mutant was used instead. The most efficient clone in terms of cross-feeding complementation was the one identified as *P. putida* BIRD-1-12F with accession number CECT 9762.

### Example 8: Obtaining a mutant microorganism resistant to sulfaguanidine derived from CECT 9761 P. putida BIRD-1-12

The CECT 9761 *P. putida* BIRD-1-12 strain obtained in Example 4 was subjected to a method of random mutagenesis similar to the one described in Example 2 but using EMS in the liquid medium (10 µL per mL of medium). For the selection of mutants, cells were sown in solid medium plates containing: 6.4 g Na₂HPO₄ x 7H₂O, 1.5 g KH₂PO₄, 0.25 g NaCl, 0.5 g NH₄Cl, 1 mL MgSO₄ 1 M, 1 mL 6 ‰ (v/v) ferric citrate, 25 mL 20% (w/v) glucose 1 g/L Bacto ™ casaminoacids, 0.1 g/L L-tyrosine , 0.1 g/L L-phenylalanine and 20 g/L sulfaguanidine.

In order to aid in the identification of positive colonies, 0.002% Congo Red was added to the medium. Plates were incubated for 1 to 2 weeks at 30 °C. Colonies resistant to sulfoguanidine were then again sown in the same medium to confirm the phenotype.

Fifty clones were selected for their ability to grow in the presence of 20 g/L sulfaguanidine.

### Example 9: Assays for determining resistance to sulfaguanidine and 5-fluoro-DL-tryptophan

The fifty colonies of mutants resistant to 20 g/L of sulfaguanidine resulting from Example 8 were sown on M9 minimal medium supplemented with 5 mg/mL, of 5-fluoro-DL-tryptophan. The sulfaguanidine-resistant clones that grew in the presence of 5 mg/mL, of 5-fluoro-DL-tryptophan were selected as candidates for overproducing tryptophan, to avoid the toxic effect of the analogue..

The most efficient sulfaguanidine-resistant strain in the presence of the analogue and that produced the greatest growth in cross-feeding assays, as performed in Example 4, was the one identified as *P. putida* BIRD-1-12S with accession number CECT 9763.

### Example 10: Use of phosphate as a phosphorus source by the selected CECT 9764 P. putida BIRD-1-21, CECT 9761 P. putida BIRD-1-12, CECT 9762 P. putida BIRD-1-12F and CECT 9763 P. putida BIRD-1-12S strains

The soluble phosphate in M9 culture medium (Example 2) was replaced with ground rock phosphate containing 10 to 30% P₂O₅ the grain size of which was less than 3 mm. Between 0.1 and 0.5 (w/v) of rock phosphate was added. After inoculation with the strains selected in Examples 4, 5, 7 and 9, it was determined that during the exponential growth phase, all the mutants bacteria doubled every 2 to 2.5 hours and the cultures reached at least 2 x 10⁹ CFU/mL.

### Example 11: Use of iron from rock phosphate phosphorus by CECT 9764 P. putida BIRD-1-21, CECT 9761 P. putida BIRD-1-12, CECT 9762 P. putida BIRD-1-12F, and CECT 9763 P. putida BIRD-1-12S strains

The soluble phosphate in M9 culture medium (Example 2) was replaced with ground rock phosphate containing trace amounts of iron in the form of Fe(III), the grain size of which was less than 3 mm. Between 0.1 and 0.5 (w/v) of rock phosphate was added. After inoculation with the strains selected in Examples 4, 7 and 9, it was determined that during the exponential growth phase, the bacteria doubled every 2 to 2.5 hours and the cultures reached more than 2 x 10⁹ CFU/mL.

### Example 12. Maintenace of PGPR properties by CECT 9764 P. putida BIRD-1-21, CECT 9761 P. putida BIRD-1-12. CECT 9762 P. putida BIRD-1-12F, and CECT 9763 P. putida BIRD-1-12S strains

To test whether the series of amino acid producing BIRD-1 variant strains conserved the wild-type properties of IAA production, of siderophore production, and organic and inorganic phosphorous solubilisation, the series of assays indicated in example 1 were carried out with the parental strain and the CECT 9764 *P. putida* BIRD-1-21, CECT 9761 *P. putida* BIRD-1-12, CECT 9762 *P. putida* BIRD-1-12F, and CECT 9763 *P. putida* BIRD-1-12S strains. In the Pikovskaya assay (test for solubilisation of inorganic phosphate) the solubilization halo produced by the parental strain after 48h incubation was about 1.5 mm. All mutant variants produced equivalent solubilisation halos that were 1.5 mm in diameter. In the CAS assay (siderophore production) the parental strain produced a siderophore halo of 3 cm in 24 h, similar to that produced by the mutant variants. IAA was recorded as turning the filter orange-red and both the parental and the mutant variants produced a similar color. Phytate solubilization was tested in phytate agar plates and the parental strain and the series of mutants, mentioned above, produced colonies that exhibited similar solubilization halos around the colonies.

### Example 13: Promoting plant growth - Stimulating the germination of horticultural plant seeds using the CECT 7415 P. putida BIRD-1 strain

Seeds of a number of horticultural plants of commercial interest for agriculture, specifically, a number of pepper, cucumber, tomato, and corn varieties, were mixed with a pure culture or with an aqueous suspension of CECT 7415 *P. putida* BIRD-1 strain (Example 1), with no stirring. At least 100 seeds of each of the selected plants treated with the CECT 7415 *P. putida* BIRD-1 strain and the same number of seeds of each of the plants treated with water (control) were then seeded in Petri dishes containing 30 g of agricultural soil.

Plates were incubated in the dark at room temperature (about 18-22°C), and the number of germinated seeds was counted after 3 days to evaluate the percentage of germination of the seeds. The results obtained were as follows:
- percentage of germination of untreated seeds: equal to or less than 65%; and
- percentage of germination of the seeds treated with *P* CECT 7415 *P. putida* BIRD-1 strain: equal to or greater than 90%.

This example clearly demonstrates that the CECT 7415 *P. putida* BIRD-1 strain stimulates the germination of horticultural plant seeds and therefore promotes the growth of said plants.

### Example 14: Promoting plant growth - Stimulating the germination of horticultural plant seeds using the CECT 9764 P. putida BIRD-1-21 strain

Seeds of a number of horticultural plants of commercial interest for agriculture, specifically, a number of peppers, cucumber, tomato, and corn varieties were mixed with a pure culture or with an aqueous suspension of CECT 9764 *P. putida* BIRD-1-21 strain (Example 4) with no stirring. At least 100 seeds of each of the selected plants treated with the CECT 9764 *P. putida* BIRD-1-21 strain and the same number of seeds of each of the plants treated with water (control) were then sown on Petri dishes containing 30 g of agricultural soil.

Plates were incubated in the dark at room temperature (at about 18-22 °C) and the number of germinated seeds was counted after 3 days to evaluate the percentage of germination of the seeds. The results obtained were as follows:
- percentage of germination of untreated seeds: equal to or less than 65%; and
- percentage of germination of the seeds treated with CECT 9764 *P. putida* BIRD-1-21 strain: equal to or greater than 92%.

This example clearly demonstrates that the CECT 9764 *P. putida* BIRD-1-21 strain stimulates the germination of horticultural plant seeds and therefore promotes the growth of said plants.

### Example 15: Promoting plant growth - Stimulating the germination of horticultural plant seeds using the CECT 9761 P. putida BIRD-1-12 strain

Seeds of a number of horticultural plants of commercial interest for agriculture, specifically, a number of pepper, cucumber, tomato, and corn varieties, were mixed with a pure culture or with an aqueous suspension of CECT 9761 *P. putida* BIRD-1-12 strain (Example 4), with no stirring. At least 100 seeds of each of the selected plants treated with the CECT 9761 *P. putida* BIRD-1-12 strain and the same number of seeds of each of the plants treated with water (control) were then seeded in Petri dishes containing 30 g of agricultural soil.

Plates were incubated in the dark at room temperature (about 18-22°C), and the number of germinated seeds was counted after 3 days to evaluate the percentage of germination of the seeds. The results obtained were as follows:
- percentage of germination of untreated seeds: equal to or less than 65%; and
- percentage of germination of the seeds treated with CECT 9761 *P. putida* BIRD-1-12 strain: equal to or greater than 93%.

This example clearly demonstrates that the CECT 9761 *P. putida* BIRD-1-12 strain stimulates the germination of horticultural plant seeds and therefore promotes the growth of said plants.

### Example 16: Promoting plant growth - Stimulating the germination of horticultural plant seeds using CECT 9762 P. putida BIRD-1-12F strain

Seeds of a number of horticultural plants of commercial interest for agriculture, specifically, a number of pepper, cucumber, tomato, and corn varieties, were mixed with a pure culture or with an aqueous suspension of CECT 9762 *P. putida* BIRD-1-12F strain (Example 7), with no stirring. At least 100 seeds of each of the selected plants treated with the CECT 9762 *P. putida* BIRD-1-12F strain and the same number of seeds of each of the plants treated with water (control) were then seeded in Petri dishes containing 30 g of agricultural soil.

Plates were incubated in the dark at room temperature (about 18-22°C), and the number of germinated seeds was counted after 3 days to evaluate the percentage of germination of the seeds. The results obtained were as follows:
- percentage of germination of untreated seeds: equal to or less than 65%; and
- percentage of germination of the seeds treated with CECT 9762 *P. putida* BIRD-1-12F strain: equal to or greater than 95%.

This example clearly demonstrates that the CECT 9762 *P. putida* BIRD-1-12F strain stimulates the germination of horticultural plant seeds and therefore promotes the growth of said plants.

### Example 17: Promoting plant growth - Stimulating the germination of horticultural plant seeds using CECT 9763 P. putida BIRD-1-12S strain

Seeds of a number of horticultural plants of commercial interest for agriculture, specifically, a number of pepper, cucumber, tomato, and corn varieties, were mixed with a pure culture or with an aqueous suspension of CECT 9763 *P. putida* BIRD-1-12S strain (Example 9), with no stirring. At least 100 seeds of each of the selected plants treated with the CECT 9763 *P. putida* BIRD-1-12S strain and the same number of seeds of each of the plants treated with water (control) were then seeded in Petri dishes containing 30 g of agricultural soil.

Plates were incubated in the dark at room temperature (about 18-22°C), and the number of germinated seeds was counted after 3 days to evaluate the percentage of germination of the seeds. The results obtained were as follows:
- percentage of germination of untreated seeds: equal to or less than 65%; and
- percentage of germination of the seeds treated with CECT 9763 *P. putida* BIRD-1-12S strain: equal to or greater than 95%.

This example clearly demonstrates that the CECT 9763 *P. putida* BIRD-1-12S strain stimulates the germination of horticultural plant seeds and therefore promotes the growth of said plants.

### Example 18: Promoting plant growth - Stimulating horticultural plant growth using CECT 7415 P. putida BIRD-1

The plant stimulating potential of CECT 7415 *P. putida* BIRD-1 strain (Example 1) on an organic substrate (peat) was evaluated. A pure culture or an aqueous suspension of CECT 7415 *P. putida* BIRD-1 was mixed with peat obtaining an active substrate (peat + strain), and then said active substrate was mixed with seeds of a number of horticultural plants of commercial interest for agriculture (pepper, cucumber, tomato, and corn) producing a mixture made up of the microorganism (CECT 7415 *P. putida* BIRD-1), the solid substrate (peat) and the seeds of the horticultural plant to be assayed. The resulting mixtures were planted in an agricultural soil and over time (depending on the plant), the percentages of germination of the seeds, stem and root lengths, the amount of fruits, and the quality of the fruits were determined. Controls were carried out by replacing the strain with water.

The percentage of germination of the seeds treated with CECT 7415 *P. putida* BIRD-1 was equal to or greater than 90%.

In successive harvests it was found that the plants that had received treatment with CECT 7415 *P. putida* BIRD-1 increased in stem length by at least 5% and in the case of the root, the number of secondary roots considerably increased, with increases from 10% to 50% of the fresh weight.

In the case of flowering and production of fruits harvest occurred sooner, in some cases weeks sooner in plants that had received treatment with CECT 7415 *P. putida* BIRD-1.

This example clearly shows that the microorganism CECT 7415 *P. putida* BIRD-1 increases stem length and the number of secondary roots during the first weeks of development of the horticultural plants leading to early harvest., and therefore promoting the growth of said plants.

### Example 19: Promoting plant growth - Stimulating horticultural plant growth using CECT 9764 P. putida BIRD-1-21

The plant stimulating potential of CECT 9764 *P. putida* BIRD-1-21 strain (Example 4) on an organic substrate (peat) was evaluated. A pure culture or an aqueous suspension of CECT 9764 *P. putida* BIRD-1-21 was mixed with peat, obtaining an active substrate (peat + strain). Said active substrate was then mixed with seeds of a number of horticultural plants of commercial interest for agriculture (pepper, cucumber, tomato, and corn), giving rise to a mixture made up of the microorganism (CECT 9764 *P. putida* BIRD-1-21), the solid substrate (peat), and the seeds of the horticultural plant to be assayed. The resulting mixtures were planted in an agricultural soil, and over time (depending on the plant) the percentages of germination of the seeds, stem and root lengths, the amount of fruits, and the quality of the fruits were determined. Controls were carried out replacing the strain with water.

The percentage of germination of the seeds treated with CECT 9764 *P. putida* BIRD-1-21 was equal to or greater than 92%.

In successive harvests it was found that plants that had received treatment with CECT 9764 *P. putida* BIRD-1-21 increased in stem length by at least between 5% and 7%, in the case of the root and the number of secondary roots also considerably increased, from 12% up to 60% of the fresh weight.

In the case of flowering and production of fruits harvest occurred sooner, in some cases weeks sooner in plants that had received treatment with CECT 9764 *P. putida* BIRD-1-21.

This example clearly shows that the microorganism CECT 9764 *P. putida* BIRD-1-21 increases stem length and the number of secondary roots during the first weeks of development of the horticultural plants, leading to early harvest and promoting the growth of said plants.

### Example 20: Promoting plant growth - Stimulating horticultural plant growth using CECT 9761 P. putida BIRD-1-12

The plant stimulating potential of CECT 9761 *P. putida* BIRD-1-12 strain (Example 4) on an organic substrate (peat) was evaluated. Briefly, a pure culture or an aqueous suspension of CECT 9761 *P. putida* BIRD-1-12 was mixed with peat, obtaining an active substrate (peat + strain), and then said active substrate was mixed with seeds of a number of horticultural plants of commercial interest for agriculture (pepper, cucumber, tomato, and corn), giving rise to a mixture made up of the microorganism (CECT 9761 *P. putida* BIRD-1-12), the solid substrate (peat), and the seeds of the horticultural plant to be assayed. The resulting mixtures were planted in an agricultural soil, and over time (depending on the plant), the percentages of germination of the seeds, stem and root lengths, the amount of fruits, and the quality of the fruits were determined. Controls were carried out by replacing the strain with water.

The percentage of germination of the seeds treated with CECT 9761 *P. putida* BIRD-1-12 was equal to or greater than 93%.

In successive harvests, it was found that the plants that had received treatment with CECT 9761 *P. putida* BIRD-1-12 increased in stem length by at least 5% to 8%, and in the case of the root, the number of secondary roots considerably increased, with increases from 13% to 65% of the fresh weight.

In the case of flowering and production of fruits, the harvest occurred sooner, in some cases weeks sooner, in those plants that had received treatment with CECT 9761 *P. putida* BIRD-1-12.

This example clearly shows that the microorganism CECT 9761 *P. putida* BIRD-1-12 increases stem length and the number of secondary roots during the first weeks of development of the horticultural plants, leads to the harvest occurring sooner, and therefore promotes the growth of said plants.

### Example 21: Promoting plant growth - Stimulating horticultural plant growth using CECT 9762 P. putida BIRD-1-12F

The plant stimulating potential of CECT 9762 *P. putida* BIRD-1-12F strain (Example 7) on an organic substrate (peat) was evaluated. Briefly, a pure culture or an aqueous suspension of CECT 9762 *P. putida* BIRD-1-12F was mixed with peat, obtaining an active substrate (peat + strain), and then said active substrate was mixed with seeds of a number of horticultural plants of commercial interest for agriculture (pepper, cucumber, tomato, and corn), giving rise to a mixture made up of the microorganism (CECT 9762 *P. putida* BIRD-1-12F), the solid substrate (peat), and the seeds of the horticultural plant to be assayed. The resulting mixtures were planted in an agricultural soil, and over time (depending on the plant), the percentages of germination of the seeds, stem and root lengths, the amount of fruits, and the quality of the fruits were determined. Controls were carried out by replacing the strain with water.

The percentage of germination of the seeds treated with CECT 9762 *P. putida* BIRD-1-12F was equal to or greater than 95%.

In successive harvests, it was found that the plants that had received treatment with CECT 9762 *P. putida* BIRD-1-12F increased in stem length by at least between 5% and 10%, and in the case of the root, the number of secondary roots considerably increased, with increases from 15% to 70% of the fresh weight.

In the case of flowering and production of fruits, the harvest occurred sooner, in some cases weeks sooner, in those plants that had received treatment with CECT 9762 *P. putida* BIRD-1-12F.

This example clearly shows that the microorganism CECT 9762 *P. putida* BIRD-1-12F increases stem length and the number of secondary roots during the first weeks of development of the horticultural plants, leads to the harvest occurring sooner, and therefore promotes the growth of said plants.

### Example 22 : Promoting plant growth - Stimulating horticultural plant growth using CECT 9763 P. putida BIRD-1-12S

The plant stimulating potential of the CECT 9763 deposited *P. putida* BIRD-1-12S strain (Example 9) on an organic substrate (peat) was evaluated. A pure culture or an aqueous suspension of CECT 9763 *P. putida* BIRD-1-12S was mixed with peat, obtaining an active substrate (peat + strain), and then the active substrate was mixed with seeds of a number of horticultural plants of commercial interest for agriculture (pepper, cucumber, tomato, and corn). This produced a mixture made up of the microorganism (CECT 9763 *P. putida* BIRD-1-12S), the solid substrate (peat), and the seeds of the horticultural plant to be assayed. The resulting mixtures were planted in an agricultural soil and over time (depending on the plant), the percentages of germination of the seeds, stem and root lengths, the amount of fruit, and the quality of the fruit were determined. Controls were carried out replacing the strain with water.

The percentage of germination of the seeds treated with CECT 9763 *P. putida* BIRD-1-12S was equal to or greater than 95%.

In successive harvests, it was found that the plants that had received treatment with CECT 9763 *P. putida* BIRD-1-12S increased in stem length by at least between 5% and 12%, and, in the case of the root, the number of secondary roots considerably increased, with increases from 15% to 70% of the fresh weight.

In the case of flowering and production of fruits, the harvest occurred sooner, in some cases weeks ealier, in those plants that had received treatment with CECT 9763 *P. putida* BIRD-1-12S.

This example clearly shows that the microorganism CECT 9763 *P. putida* BIRD-1-12S increases stem length and the number of secondary roots during the first weeks of development of the horticultural plants, leading to ealy harvest and promoting the growth of the aforementioned plants.

### Example 23: Promoting plant growth - Stimulating the germination of grass-type seeds using the CECT 9764 P. putida BIRD-1-21 strain

The assay described in Example 14 was repeated using various types of grass seeds such as *Dichondria repens, Ryegrass, Agrostis stolonifera, Poa pratensis,* and different types of fescue, etc. The results showed that the percentage of germination of the seeds treated with CECT 9764 *P. putida* BIRD-1-21 (Example 4) was 100%, whereas in the case of the untreated seeds the percentage of germination was equal to or less than 90%. This example clearly shows that the CECT 9764 *P. putida* BIRD-1-21 strain stimulates the germination of grass seeds.

### Example 24: Promoting plant growth - Stimulating the germination of grass-type seeds using the CECT 9761 P. putida BIRD-1-12 strain

The assay described in Example 15 was repeated using various types of grass seeds such as *Dichondria repens, Ryegrass, Agrostis stolonifera, Poa pratensis,* various types of fescue, etc. The results showed that the percentage of germination of the seeds treated with CECT 9761 *P. putida* BIRD-1-12 (Example 4) was 100%, whereas in the case of the untreated seeds, the percentage of germination was equal to or less than 90%. This example clearly shows that the CECT 9761 *P. putida* BIRD-1-12 strain stimulates the germination of grass seeds.

### Example 25: Promoting plant growth - Stimulating the germination of grass-type seeds using the CECT 9762 P. putida BIRD-1-12F strain

The assay described in Example 16 was repeated using various types of grass seeds such as *Dichondria repens, Ryegrass, Agrostis stolonifera, Poa pratensis,* various types of fescue, etc. The results showed that the percentage of germination of the seeds treated with CECT 9762 *P. putida* BIRD-1-12F (Example 7) was 100%, whereas in the case of the untreated seeds, the percentage of germination was equal to or less than 90%. This example clearly shows that the CECT 9762 *P. putida* BIRD-1-12F strain stimulates the germination of grass seeds.

### Example 26: Promoting plant growth - Stimulating the germination of grass-type seeds using the CECT 9763 P. putida BIRD-1-12S strain

The assay described in Example 17 was repeated using various types of grass seeds such as *Dichondria repens, Ryegrass, Agrostis stolonifera, Poa pratensis,* various types of fescue, etc. The results showed that the percentage of germination of the seeds treated with CECT 9763 *P. putida* BIRD-1-12S (Example 7) was 100%, whereas in the case of the untreated seeds the percentage of germination was equal to or less than 90%. This example clearly shows that the CECT 9763 *P. putida* BIRD-1-12S strain stimulates the germination of grass seeds.

### Example 27: Promoting plant growth. Stimulating grass growth using the CECT 9764 P. putida BIRD-1-21 strain

The assay described in Example 19 was repeated but with the grass seeds mentioned in Example 22, obtaining favourable results in all the cases in which the seed was associated with the previously described active solid support [peat + *P. putida* BIRD-1-21 (CECT 9764)]. An increase in the number of germinated seeds and earlier plant development was observed along with a parallel increase in the percentage of plant cover, which was 100% in the case of the sown soil covered with grass seeds and the active solid support, and less than 85% in the case of the soil sown with the grass seeds and the solid support without the microorganism.

Again, this example clearly shows that the microorganism *P. putida* BIRD-1-21 (CECT 9764) stimulates the germination of grass seeds.

### Example 28: Promoting plant growth. Stimulating grass growth using the CECT 9761 P. putida BIRD-1-12 strain

The assay described in Example 19 was repeated but with the grass seeds mentioned in Example 23, obtaining favourable results in all the cases in which the seed was associated with the previously described active solid support [peat + *P. putida* BIRD-12 (CECT 9761)]. An increase in the number of germinated seeds and earlier plant development was observed with a parallel increase in the percentage of plant cover, which was 100% in the case of the sown soil covered with grass seeds and the active solid support, and less than 85% in the case of the soil sown with the grass seeds and the solid support without the microorganism.

Again, this example clearly shows that the microorganism *P. putida* BIRD-1-12 (CECT 9761) stimulates the germination of grass seeds.

### Example 29: Promoting plant growth. Stimulating grass growth using the CECT 9762 P. putida BIRD-1-12F strain

The assay described in Example 20 was repeated but with the grass seeds mentioned in Example 24, obtaining favourable results in all the cases in which the seed was associated with the previously described active solid support [peat + *P. putida* BIRD-12F (CECT 9762)]. An increase in the number of germinated seeds and earlier plant development was observed with a parallel increase in the percentage of plant cover, which was 100% in the case of the sown soil covered with grass seeds and the active solid support and less than 85% in the case of the soil sown with the grass seeds and the solid support without a microorganism.
Again, this example clearly shows that the microorganism *P. putida* BIRD-1-12F (CECT 9762) stimulates the germination of grass seeds.

### Example 30: Promoting plant growth. Stimulating grass growth using the CECT 9763 P. putida BIRD-1-12S strain

The assay described in Example 21 was repeated but with the grass seeds mentioned in Example 25. Favourable results were obtained in all the cases in which the seed was associated with the previously described active solid support [peat + *P. putida* BIRD-1-12S (CECT 9763)]. An increase in the number of germinated seeds and earlier plant development was observed with, an parallel increase in the percentage of plant cover, which was 100% in the case of the sown soil covered with grass seeds and the active solid support and less than 85% in the case of the soil sown with the grass seeds and the solid support without a microorganism.
Again, this example clearly shows that the microorganism *P. putida* BIRD-1-12S (CECT 9763) stimulates the germination of grass seeds.

### Example 31: Liquid fertiliser supplement using the CECT 9764 P. putida BIRD-1-21 strain

A pure culture or an aqueous suspension of CECT 9764 *P. putida* BIRD-1-21 (Example 4) was mixed with various liquid fertiliser formulations, such as the products Herofulvat®, Herocotton® Starter, or Herovital® and other similar products supplied by the manufacturer, i.e., Herogra. The concentration of CECT 9764 *P. putida* BIRD-1-21 was in all cases at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle were obtained, i.e., the inoculated horticultural species flowered and fruited at least 20 days sooner.

### Example 32: Liquid fertiliser supplement using the CECT 9761 P. putida BIRD-1-12 strain

A pure culture or an aqueous suspension of CECT 9761 *P. putida* BIRD-1-12 (Example 4) was mixed with various liquid fertiliser formulations, such as the products Herofulvat®, Herocotton® Starter, or Herovital® and other similar products supplied by the manufacturer, i.e. Herogra. The concentration of CECT 9761 *P. putida* BIRD-1-12 was, in all cases, at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle were obtained, i.e., the inoculated horticultural species flowered and fruited at least 20 days sooner.

### Example 33: Liquid fertiliser supplement using the CECT 9762 P. putida BIRD-1-12F strain

A pure culture or an aqueous suspension of CECT 9762 *P. putida* BIRD-1-12F (Example 7) was mixed with various liquid fertiliser formulations, such as the products Herofulvat®, Herocotton® Starter, or Herovital® and other similar products supplied by the manufacturer, i.e. Herogra. The concentration of CECT 9762 *P. putida* BIRD-1-12F was, in all cases, at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle were obtained, i.e., the inoculated horticultural species flowered and fruited at least 20 days sooner.

### Example 34: Liquid fertiliser supplement using the CECT 9763 P. putida BIRD-1-12S strain

A pure culture or an aqueous suspension of CECT 9763 *P. putida* BIRD-1-12S (Example 9) was mixed with various liquid fertiliser formulations, such as Herofulvat®, Herocotton® Starter, or Herovital® and other similar products supplied by the manufacturer, i.e., Herogra. The concentration of CECT 9763 *P. putida* BIRD-1-12S was, in all cases, at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle were obtained, i.e., the inoculated horticultural species flowered and fruited at least 20 days sooner.

### Example 35: Seed supplement using the CECT 9764 P. putida BIRD-1-21 strain

Cells of the CECT 9764 *P. putida* BIRD-1-21 (Example 4) were adhered to an organic polymer, such as alginate or carbopol gel [mixture of viscous mucilage (CARBOPOL 940) and triethanolamine] and put in contact with various seeds both of agricultural interest, such as eggplants, zucchinis, beans, green beans, melon, tomatoes, etc., and of ornamental interest, such as lavender plants, petunia plants, chrysanthemum plants, etc. The concentration of CECT 9764 *P. putida* BIRD-1-21 was at least 10⁵ cfu/g, obtaining satisfactory results in all cases (at least a 10% size increase in both the aerial part and the root, better plant colour, and earlier flowering and fruiting).

### Example 36: Seed supplement using the CECT 9761 P. putida BIRD-1-12 strain

Cells of the CECT 9761 *P. putida* BIRD-1-12 (Example 4) were adhered to an organic polymer, such as alginate and carbopol gel [mixture of viscous mucilage (CARBOPOL 940) and triethanolamine] and put in contact with various seeds both of agricultural interest, such as eggplants, zucchinis, beans, green beans, melon, tomatoes, etc., and of ornamental interest, such as lavender plants, petunia plants, chrysanthemum plants, etc. The concentration of CECT 9761 *P. putida* BIRD-1-12 was at least 10⁵ cfu/g, obtaining satisfactory results in all cases of at least a 10% size increase in both the aerial part and the root, better plant colour, and earlier flowering and fruiting.

### Example 37: Seed supplement using the CECT 9762 P. putida BIRD-1-12F strain

Cells of the CECT 9762 *P. putida* BIRD-1-12F strain (Example 7) were adhered to an organic polymer, such as alginate and carbopol gel [mixture of viscous mucilage (CARBOPOL 940) and triethanolamine] and put in contact with various seeds both of agricultural interest, such as eggplants, zucchinis, beans, green beans, melon, tomatoes, etc., and of ornamental interest, such as lavender plants, petunia plants, chrysanthemum plants, etc. The concentration of CECT 9762 *P. putida* BIRD-1-12F strain was at least 10⁵ cfu/g, obtaining satisfactory results in all cases of at least a 10% size increase in both the aerial part and the root, better plant colour, and earlier flowering and fruiting.

### Example 38: Seed supplement using the CECT 9763 P. putida BIRD-1-12S strain

Cells of the CECT 9763 *P. putida* BIRD-1-12S strain (Example 9) were adhered to an organic polymer, such as alginate or carbopol gel [mixture of viscous mucilage (CARBOPOL 940) and triethanolamine] and put in contact with various seeds both of agricultural interest, such as eggplants, zucchinis, beans, green beans, melon, tomatoes, etc., and of ornamental interest, such as lavender plants, petunia plants, chrysanthemum plants, etc. The concentration of CECT 9763 *P. putida* BIRD-1-12S strain was at least 10⁵ cfu/g obtaining satisfactory results in all cases (at least a 10% size increase in both the aerial part and the root, better plant colour, and earlier flowering and fruiting).

### Example 39: Liquid fertiliser supplement using the CECT 9764 P. putida BIRD-1-21 strain

A pure culture or an aqueous suspension of CECT 9764 *P. putida* BIRD-1-21 (Example 4) was mixed with various liquid fertiliser formulations, such as the products Herofulvat®, Herocotton® Starter, or Herovital® and other similar products supplied by the manufacturer, i.e. Herogra. The concentration of CECT 9764 *P. putida* BIRD-1-21 was, in all cases, at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle was observed, i.e., the inoculated horticultural species flowered and fruited at least 20 days sooner.

### Example 40: Liquid fertiliser supplement using the CECT 9761 P. putida BIRD-1-12 strain

A pure culture or an aqueous suspension of CECT 9761 *P. putida* BIRD-1-12 (Example 4) was mixed with various liquid fertiliser formulations, such as the products Herofulvat®, Herocotton® Starter, or Herovital® and other similar products supplied by the manufacturer, i.e., Herogra. The concentration of CECT 9761 *P. putida* BIRD-1-12 was in all cases at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle was observed, i.e., the inoculated horticultural species flowered and fruited at least 20 days sooner.

### Example 41: Liquid fertiliser supplement using the CECT 9762 P. putida BIRD-1-12F strain

A pure culture or an aqueous suspension of CECT 9762 *P. putida* BIRD-1-12F (Example 7) was mixed with various liquid fertiliser formulations, such as the products Herofulvat®, Herocotton® Starter, or Herovital® and other similar products supplied by the manufacturer, i.e., Herogra. The concentration of CECT 9762 *P. putida* BIRD-1-12F was in all cases at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle was observed, i.e., the inoculated horticultural species flowered and fruited at least 20 days sooner.

### Example 42: Liquid fertiliser supplement using the CECT 9763 P. putida BIRD-1-12S strain

A pure culture or an aqueous suspension of CECT 9763 *P. putida* BIRD-1-12S (Example 9) was mixed with various liquid fertiliser formulations, such as the products Herofulvat®, Herocotton® Starter, or Herovital® and other similar products supplied by the manufacturer, i.e., Herogra. The concentration of CECT 9763 *P. putida* BIRD-1-12S was in all cases at least 10⁵ cfu/mL. The liquid fertiliser supplemented with said microorganism was used for stimulating plant growth of a number of horticultural plants of commercial interest, such as eggplants, zucchinis, beans, green beans, melon, tomatoes, etc., obtaining satisfactory results in all cases. Specifically, a growth increase of at least 30% and an earlier plant cycle was observed, i.e., the inoculated horticultural species flowered and fruited at least 20 days sooner.

### Example 43: Analysis of tryptophan from the culture broth of CECT 7415 P. putida BIRD-1, CECT 9764 P. putida BIRD1-21, CECT 9761 P. putida BIRD-1-12. CECT 9762 P. putida BIRD-1-12F, and CECT 9763 P. putida BIRD-1-12S

Two hundred mL of each of the culture broths of strains CECT 7415 *P. putida* BIRD-1, CECT 9764 *P. putida* BIRD1-21, CECT 9761 *P. putida* BIRD1-12, CECT 9762 *P. putida* BIRD1-12F, and CECT 9763 *P.putida* BIRD-1-12S were centrifuged for 10 minutes at 4 °C and the resulting supernatant was filtered twice though 0.22µm sterile filters.. A mixture v/v of the supernatants and cold acetone was then mixed with a vortex. This step eliminates residual proteins. Said mixture was again centrifuged for 60 minutes at 2,000 rpm at 4 °C. The resulting supernatants were filtered through 0.22 µm sterile filters.

The filtered mixtures were evaporated using a speed vac and preserved at -20° C until measured. The resulting pellets are equivalent to 1 mL of supernatant of each of the culture of the strains of the invention. Pellets were suspended in a final volume of 100 µL of milliQ water. Finally, 2 µL of the sample was used for measurements and the nature and concentration of tryptophan present in the suspension was determined by means of XGVO(LC-MS-MS)

The results obtained clearly showed that, while the parent strain CECT 7415 *P. putida* BIRD-1 does not produce L-tryptophan, the *P. putida* strains BIRD-1-12, 1-12F, 1-21 and 1-12S (CECT 9761, CECT 9762, CECT 9764 and CECT 9763, produce L-tryptophan at concentrations ranging between 1 and 100 µM according to the growth phase of the microorganism).

### Example 44 : Analysis of the presence of amino acids other than tryptophan in the culture broth of CECT 7415 P. putida BIRD-1, CECT 9764 P. putida BIRD1-21, CECT 9761 P. putida BIRD-1-12, CECT 9762 P. putida BIRD-1-12F and CECT 9763 P. putida BIRD-1-12S

Two hundred mL of each of the culture broths of said strains CECT 7415 *P. putida* BIRD-1, CECT 9764 *P. putida* BIRD-1-21, CECT 9761 *P. putida* BIRD-1-12, CECT 9762 *P. putida* BIRD1-12F, and CECT 9763 *P. putida* BIRD1-12S were centrifuged for 10 minutes at 4°C and the resulting supernatant was then filtered twice using 0.22 µm sterile filters. A mixture v/v of the supernatants and cold acetone was then mixed with a vortex. This step eliminates residual proteins. Said mixture was again centrifuged for 60 minutes at 2,000 rpm at 4 °C. The resulting supernatants were filtered through 0.22 µm sterile filters.

The filtered mixtures were evaporated using a speed vac and preserved at -20° C until measured. The resulting pellets are equivalent to 1 mL of supernatant of each of the culture of the strains of the invention. Pellets were suspended in a final volume of 100 µL of buffer and derivatizing agent. Finally, 1 µL of the sample was used for measurements. Quantification of the amino acids (other than tryptophan) was performed using ACCq-Tag Ultra Method Derivatization Chemistry with AccQ-Tag Ultra Derivatization Kit from Waters Corporation (FLR detector).

The results obtained are shown in Table 1. It can be seen that, while the parent strain CECT 7415 *P. putida* BIRD-1 and BIRD-1-21 CECT 9764 produce basal arginine, phenylalanine, and methionine, mutant CECT 9761 *P. putida* BIRD-1-12 produces in addition to tryptophan arginine, CECT 9762 *P. putida* BIRD-1-12F, produces phenylalanine in addition to tryptophan, and CECT 9763 *P. putida* BIRD-1-12S produces in addition to tryptophan, phenylalanine, arginine and methionine. Concentrations are given in micromolar after 18h culture at 30°C in the above described minimal medium.

| | Phenylalanine | Arginine | Methionine |
|---|---|---|---|
| BIRD-1 | 29 | 54 | 27 |
| BIRD-1-21 | 35 | 59 | 10 |
| BIRD-1-12 | 34 | 106 | 26 |
| BIRD-1-12F | 177 | 57 | 18 |
| BIRD-1-12S | 60 | 149 | 72 |

### Example 45: Ability of CECT 9764 P. putida BIRD-1-21, CECT 9761 P. putida BIRD-1-12, CECT 9762 P. putida BIRD-1-12F, and CECT 9763 P. putida BIRD-1-12S to form biofilms on abiotic surfaces in order to form biofilms on different types of surfaces

Cultures of the CECT 9764 *P. putida* BIRD-1-21, CECT 9761 *P. putida* BIRD1-12, CECT 9762 *P. putida* BIRD1-12F, and CECT 9763 *P. putida* BIRD1-12S strains were incubated *in vitro* for 12 h and then used to inoculate, at a 1:20 ratio, 50 mL of LB medium or M9 minimal medium supplemented with glucose, in polystyrene microtitration plates. After 4 h of incubation at 30°C, the wells were washed with deionized water, and 100 µL of a 1% crystal violet solution (w/v) were added to each well. The plates were incubated at room temperature for 15 min, after which the wells were thoroughly washed, and biofilm formation was quantified. For this purpose, the residue stained blue was solubilized by adding ethanol (200 µL, two fold) to each well. This solution was transferred to an Eppendorf tube, and 600 µL of distilled water were added. Then absorbance at 600 nm was measured in a Shimadzu UV-1700 spectrophotometer. While in a control without cells, absorbance of the supernatant at 600 nm was below 0.1, in the case of the wild-type or any of the mutant variants, absorbance at 600 nm was in the range of 0.8 to 1.0

Similar assays with a positive result were performed to verify biofilm formation on other surfaces, such as polypropylene (Eppendorf tubes), and borosilicate (glass tubes).

All the strains form biofilms reaching densities of at least 10⁸ cfu/cm².

### Example 46: Ability of CECT 9764 P. putida BIRD-1-21, CECT 9761 P. putida BIRD-1-12. CECT 9762 P. putida BIRD1-12F, and CECT 9763 P. putida BIRD-1-12S to form biofilms on the surface of roots of horticultural plants and their involvement in promoting plant growth and protection against adverse agents

To verify biofilm formation on the surface of roots of plants of agricultural interest (tomato and corn), seeds having their surface sterilised previously were submerged in a culture of each of CECT 9764 *P. putida* BIRD-1-21, CECT 9761 *P. putida* BIRD-1-12, CECT 9762 *P. putida* BIRD-1-12F, and CECT 9763 *P. putida* BIRD-1-12S (at 10⁸ cfu/mL). After 30 min, the seeds were washed with sterile distilled water and then placed in Petri dishes with MS medium (Murashige & Skoog, 1962, Physiologia Plantarum 15:473-97), and they were incubated at 25°C with a photoperiod of 16 hours of light and 8 hours of darkness, until reaching a minimum length of 3 cm. Germination of the seeds was monitored under scanning electron microscopy, with biofilm formation being observed in all cases.

The rest of the seedlings were transplanted into plant bed trays to which an organic substrate (peat) had previously been added. Growth of the rest of the plants inoculated with the above strains was observed in comparison to the respective non-inoculated controls for at least another 15 days. After that time, at least a 15 to 30% increase in fresh weight of the plants that had previously been inoculated with any of the above *P. putida* strains used in the assay was found. In order to verify biofilm formation, after washing the roots, the adhered cells were separated from the root by sonication in an ultrasonic bath. At least 10⁹ cfu were recovered per cm² from the roots.

The formation and maintenance of the biofilm in the root formed by each of the above strains of *P. putida* completely occupies said ecological niche, preventing colonization of another type of rhizospheric microorganisms that may be harmful to plant development. Moreover, it has been described that biofilm formation by plant growth promoting microorganisms on the roots of plants provides them with greater protection against abiotic stress situations (high or low temperatures, drought, etc.).

## Claims

1. A variant strain of *Pseudomonas putida* BIRD-1 with accession number CECT 7415, which variant strain produces at least one plant growth-stimulating compound and/or solubilises insoluble phosphate and/or iron.

2. The variant strain according to claim 1, wherein the at least one plant growth-stimulating compound is an L- amino acid.

3. The variant strain according to claim 2, wherein the L- amino acid is selected from the group consisting of L-Tryptophan, L-Phenylalanine, L-Arginine and L-Methionine

4. The variant strain according to claim 3, wherein the L- amino acid is L-Tryptophan and wherein the strain further produces at least one L- amino acid selected from the group consisting of L-Phenylalanine, L-Arginine and L-Methionine.

5. The strain according to any of claims 1 to 4, wherein said variant is selected from the group consisting of *Pseudomonas putida* BIRD-1-12 with accession number CECT 9761, *Pseudomonas putida* BIRD-1-12F with accession number CECT 9762, *Pseudomonas putida* BIRD-1-12S with accession number CECT 9763, *Pseudomonas putida* BIRD-1-21 with accession number CECT 9764 and variants thereof which maintain the capacity to produce at least one plant growth-stimulating compound and/or to solubilise insoluble phosphate and/or iron.

6. A supernatant of a culture of at least one variant strain according to any of claims 1 to 5.

7. A fertiliser, seed, root ball, substrate, solid support, soil or field comprising at least one variant strain according to any of claims 1 to 5, or a supernatant according to claim 6.

8. Method for stimulating plant growth, comprising the step of applying an effective amount of at least one variant strain according to any of claims 1 to 5, or a supernatant according to claim 6, or a fertiliser, substrate, solid support or soil according to claim 7, to the plant or, alternatively, the step of planting a seed or a root ball according to claim 7.

9. Method for restoring the normal state of a plant after suffering environmental stress, comprising the step of applying an effective amount of at least one variant strain according to any of claims 1 to 5, or a supernatant according to claim 6, or a fertiliser, substrate, solid support or soil according to claim 7, to the plant or, alternatively, the step of planting a seed or a root ball according to claim 7.

10. Method for solubilising an insoluble phosphate and/or insoluble iron in a solid substrate or field, comprising the step of applying at least one variant strain according to any of claims 1 to 5, or a fertiliser, substrate, solid support or soil according to claim 7, to the plant or, alternatively, the step of planting a seed or a root ball according to claim 7 on the solid substrate or field.

11. Method for obtaining a strain with capacity to produce at least one plant growth-stimulating compound and/or to solubilise insoluble phosphate and/or iron comprising the steps of:
(i) mutagenizing *P. putida* BIRD-1 strain with accession number CECT 7415 or the variant strain according to any of claims 1 to 5 in the presence of an analogue of the plant growth-stimulating compound and
(ii) selecting a strain capable of growing in the presence of an analogue of the growth-stimulating compound.

12. The method according to claim 11, wherein the step of mutagenesis comprises putting the *P. putida* BIRD-1 strain with accession number CECT 7415 or the variant strain according to any of claims 1 to 5 in contact with a mutagenic agent in a culture medium comprising a carbon source, a nitrogen source, a phosphorus source, micronutrients, and an analogue of the growth-stimulating compound.

13. The method according to any of claims 11 or 12, wherein the mutagenizing step is by means of an alkylating agent or by exposure to UV light

14. A strain obtainable by the method according to any of claims 11 to 13.

15. Use of the variant strain according to any of claims 1 to 5, or the supernatant according to claim 6, or the fertiliser, seed, root ball, substrate, active solid support, or soil according to claim 7, for stimulating plant growth and/or for restoring the normal state of a plant after suffering environmental stress and/or for obtaining a strain with capacity to produce at least one plant growth-stimulating compound and/or to solubilise insoluble phosphate and/or iron.
